(19) Europäisches Patentamt European Patent Office Office européen des brevets

(11) **EP 3 212 079 B1**

(12) **EUROPEAN PATENT SPECIFICATION**

(45) Date of publication and mention of the grant of the patent:
**12.06.2019 Bulletin 2019/24**

(21) Application number: **15854089.8**

(22) Date of filing: **23.04.2015**

(51) Int Cl.:
*A61B 5/055* (2006.01)  *A61B 90/00* (2016.01)
*A61B 6/03* (2006.01)  *A61B 6/00* (2006.01)
*G01R 33/28* (2006.01)  *A61B 5/00* (2006.01)
*A61B 3/113* (2006.01)  *A61B 5/11* (2006.01)
*A61B 8/00* (2006.01)

(86) International application number:
**PCT/KR2015/004031**

(87) International publication number:
**WO 2016/068420 (06.05.2016 Gazette 2016/18)**

(54) **MEDICAL IMAGING APPARATUS**

MEDIZINISCHE BILDGEBUNGSVORRICHTUNG

APPAREIL D'IMAGERIE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2014 KR 20140146106**

(43) Date of publication of application:
**06.09.2017 Bulletin 2017/36**

(73) Proprietor: **Samsung Electronics Co., Ltd.**
**Suwon-si, Gyeonggi-do 443-742 (KR)**

(72) Inventors:
• **GULAKA, Praveen**
**Suwon-si**
**Gyeonggi-do 443-727 (KR)**
• **JO, Hyun Hee**
**Osan-si**
**Gyeonggi-do 447-768 (KR)**
• **JO, Jae Moon**
**Seongnam-si**
**Gyeonggi-do 463-858 (KR)**
• **CHOI, Yang Lim**
**Seongnam-si**
**Gyeonggi-do 463-743 (KR)**

(74) Representative: **Grootscholten, Johannes A.M. et al**
**Arnold & Siedsma**
**Bezuidenhoutseweg 57**
**2594 AC The Hague (NL)**

(56) References cited:
WO-A1-2008/120152      JP-A- 2001 314 391
JP-A- 2004 180 834      JP-A- 2014 195 616
US-A- 5 134 373          US-A1- 2012 143 040
US-A1- 2012 302 867    US-A1- 2013 208 249
US-A1- 2013 218 004    US-A1- 2014 125 337
US-A1- 2014 125 337

**Description**

Embodiments of the present disclosure relate to a medical imaging apparatus.

With growing interest in healthcare, active research on medical imaging apparatuses is in progress. As examples of medical imaging apparatuses, there may be ultrasound imaging apparatuses, X-ray imaging apparatuses, Computed Tomography (CT) devices, Positron Emission Tomography (PET) devices, Magnetic Resonance Image (MRI) devices, etc.

The ultrasound imaging apparatus is a device for generating an image of an internal part of a subject, such as a cross section of soft tissues or blood flow, by irradiating ultrasounds from the surface of the subject and detecting reflected ultrasounds. The X-ray imaging apparatus is a device for obtaining an image of an internal part of a subject by irradiating X-rays to the subject and detecting X-rays transmitted through the subject, and the CT device obtains a tomogram image of a subject by having X-rays pass through the subject at different angles. The PET device is a device for producing a tomogram image by injecting a positron-emitting radionuclide into a subject and detecting gamma rays emitted from the positron. The MRI device is a device for imaging an internal part of a subject by supplying energy at a certain frequency while applying a magnetic field to the nucleus of hydrogen of a subject, and converting energy emitted from the nucleus.

Sometimes scanning with the medical imaging apparatus requires long time to obtain an image of an internal part of a subject. For example, it may take at least 20 minutes to 1 hour or more for MRI scanning, depending on e.g., portions to be scanned and MRI image types. Long time scanning may bore the patient (subject), causing a movement of the subject during the scanning process. Movements of the subject may lead to quality degradation of the scanned image of the subject, and accordingly, a need exists for development of an apparatus or method for relieving boredom of the subject during the scanning process.

Here, US-2013/208249 and US-2012/143040 are acknowledged to disclose stationary screen units, while US-2014/125337 displays images from a projector onto the interior of a gantry, while US-5143373 disloses a tiltable mirror, wherein at least the features in the characterizing portion of the appended independent claim is novel and impart inventive step on the disclosure as a whole in order to provide out-of bore display.

The present disclosure provides a medical imaging apparatus.

In accordance with an aspect of the present disclosure, a medical imaging apparatus is provided in accordance with appended independent claim 1.

**Advantageous Effects of Invention**

In accordance with embodiments of the medical imaging apparatus, various content may be provided for a person subject to scanning to relieve his/her boredom or inconvenience during the scanning process.

**Brief Description of Drawings**

The above and other features and advantages of the present disclosure will become more apparent by describing in detail exemplary embodiments thereof with reference to the attached drawings in which:

FIG. 1 is a block diagram of a medical imaging apparatus, according to an embodiment of the present disclosure;

FIGS. 2A and 2B show the exterior of a medical imaging apparatus, according to an embodiment of the present disclosure;

FIGS. 3A and 3B show the exterior of a medical imaging apparatus, according to another embodiment of the present disclosure;

FIG. 4 shows a space where a subject is lying down, which is classified into X, Y, and Z axes;

FIG. 5 shows structures of a gantry and a gradient coil unit;

FIG. 6 shows a pulse sequence related to respective operation of gradient coils that constitute a gradient coil unit;

FIG. 7 shows a schematic arrangement of an image projector, according to an embodiment of the present disclosure;

FIG. 8 shows an image projector mounted on a supporter, according to an embodiment of the present disclosure;

FIG. 9 shows a schematic arrangement of an image projector, according to anther embodiment of the present disclosure;

FIG. 10 is a block diagram of a beam projector, according to an embodiment of the present disclosure;

FIG. 11 shows a schematic arrangement of an image projector, according to another embodiment of the present disclosure;

FIG. 12 is a diagram for explaining a screen unit, according to an embodiment of the present disclosure;

FIG. 13 shows an occasion where a push/pull screen unit is mounted on a second gantry;

FIG. 14 shows a medical imaging apparatus having a first gantry on which a push/ pull screen unit is mounted, and a plurality of image projectors;

FIG. 15 shows a medical imaging apparatus having a second gantry on which a plurality of push/pull screen units are mounted, and a plurality of image projectors;

FIG. 16 is a diagram for explaining a screen unit, according to another embodiment of the present disclosure;

FIG. 17 shows an occasion where a rotational screen unit is mounted on a second gantry;

FIG. 18 shows a medical imaging apparatus having a first gantry on which a rotational screen unit is mounted, and a plurality of image projectors;

FIG. 19 shows a medical imaging apparatus having a second gantry on which a plurality of rotational screen units are mounted, and a plurality of image projectors;

FIG. 20 is a diagram for explaining a screen unit, according to another embodiment of the present disclosure;

FIG. 21 shows an occasion where a fixed screen unit is arranged above a second gantry;

FIG. 22 shows a medical imaging apparatus having a fixed screen unit and a plurality of image projectors;

FIG. 23 shows a medical imaging apparatus having a plurality of fixed screen units and a plurality of image projectors;

FIG. 24 is a control block diagram of a medical imaging apparatus, according to another embodiment of the present disclosure;

FIG. 25 shows a first gantry on which a push/pull screen unit and an image sensor are mounted;

FIG. 26 shows a first gantry on which a rotational screen unit and an image sensor are mounted;

FIG. 27 shows a first gantry with a fixed screen unit arranged above the first gantry and an image sensor;

FIG. 28 shows a medical imaging apparatus including a first gantry and a weight sensor, according to an embodiment of the present disclosure;

FIG. 29 shows a medical imaging apparatus including a first gantry and a weight sensor, according to another embodiment of the present disclosure;

FIG. 30 shows a second gantry on which a push/pull screen unit and an image sensor are mounted, according to an embodiment of the present disclosure;

FIG. 31 shows a second gantry on which an image sensor is mounted, according to an embodiment of the present disclosure;

FIG. 32 shows a medical imaging apparatus including a second gantry, a switch, and a weight sensor, according to an embodiment of the present disclosure;

FIG. 33 shows a medical imaging apparatus including a switch and a weight sensor, according to an embodiment of the present disclosure;

FIG. 34 shows a medical imaging apparatus including a second gantry, a photo sensor, and a weight sensor, according to an embodiment of the present disclosure;

FIG. 35 shows a medical imaging apparatus including a photo sensor and a weight sensor, according to an embodiment of the present disclosure;

FIG. 36 shows a medical imaging apparatus including an eyeball tracking sensor and a push/pull screen unit, according to an embodiment of the present disclosure;

FIG. 37 shows a medical imaging apparatus including an eyeball tracking sensor and a rotational screen unit, according to an embodiment of the present disclosure;

FIG. 38 shows a medical imaging apparatus including an eyeball tracking sensor and a fixed screen unit, according to an embodiment of the present disclosure;

FIG. 39 is a block diagram of a medical imaging apparatus, according to another embodiment of the present disclosure;

FIGS. 40 to 42 show a medical imaging apparatus including a push/pull screen unit and a rotation guard, according to an embodiment of the present disclosure;

FIG. 43 shows a medical imaging apparatus including a rotational screen unit and a rotation guard, according to an embodiment of the present disclosure; and

FIG. 44 shows diagrams for explaining rotation of a rotation guard and screen unit, according to an embodiment of the present disclosure.

Throughout the drawings, like reference numerals will be understood to refer to like parts, components, and structures.

## Best Mode for Carrying out the Invention

Embodiments and features as described and illustrated in the present disclosure are only preferred examples, and various modifications thereof may also fall within the scope of the disclosure. Embodiments of the present disclosure not falling under the scope of independent claim 1 do not form part of the invention.

A medical imaging apparatus in accordance with various embodiments of the present disclosure will now be described in detail with reference to accompanying drawings. Like reference numerals indicate like elements throughout the specification.

A medical imaging apparatus may be an ultrasound imaging apparatus, an X-ray imaging apparatus, a Computed Tomography (CT) device, a Positron Emission Tomography (PET) device, a Magnetic Resonance Image (MRI) device, or the like. It is, however, not limited thereto and may include any medical imaging apparatus capable of imaging an internal part of a patient (hereinafter, referred to as a subject). For convenience of explanation, the medical imaging apparatus will be applied to an MRI device.

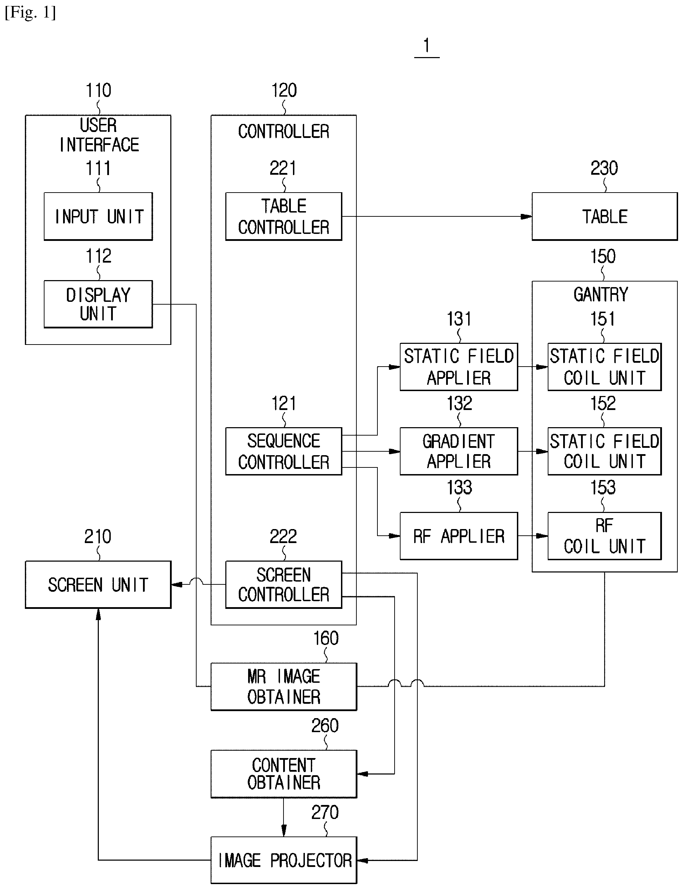

FIG. 1 is a block diagram of a medical imaging apparatus, according to an embodiment of the present disclosure.

Referring to FIG. 1, a medical imaging apparatus 1 may include a user interface 110 for providing a user interface, a table 230 for carrying a subject, a gantry 150 for producing a magnetic field and receiving a magnetic resonance signal produced from the subject, a Magnetic Resonance (MR) image obtainer 160 for performing various processes on the magnetic resonance signal to obtain a magnetic resonance image, a content obtainer 260 for obtaining various content to be provided for the subject, an image projector 270 for projecting images contained in the content, a screen unit 210 to display the projected image, and a controller 120 for controlling overall operation of the medical imaging apparatus 1. The subject as herein used may be a living body of a human or animal, and any object whose internal structure may be imaged by the medical imaging apparatus 1. However, for convenience of explanation, the subject will be assumed herein to be a body of a person.

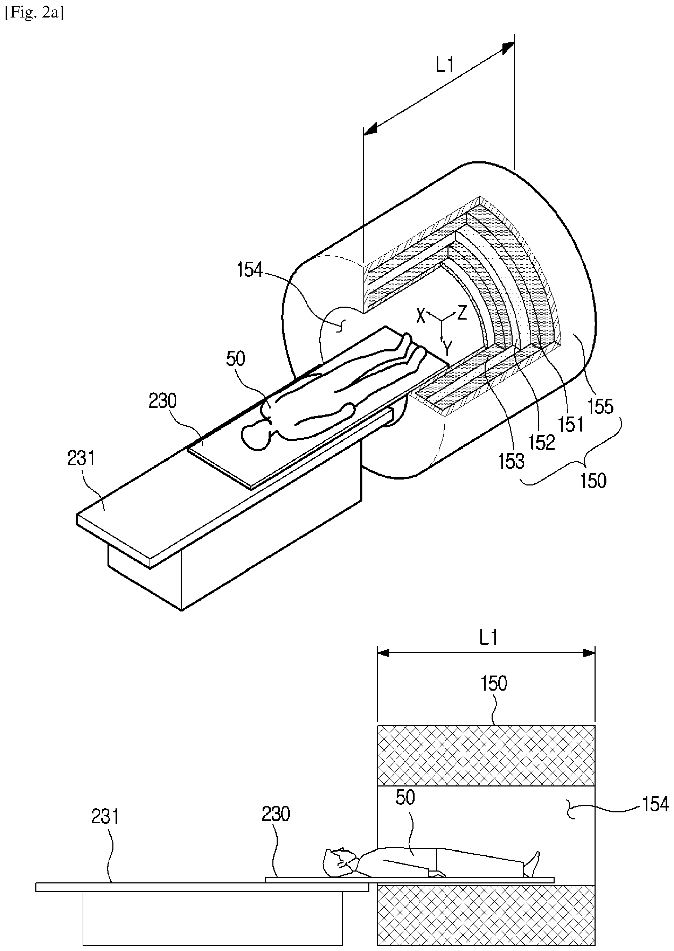

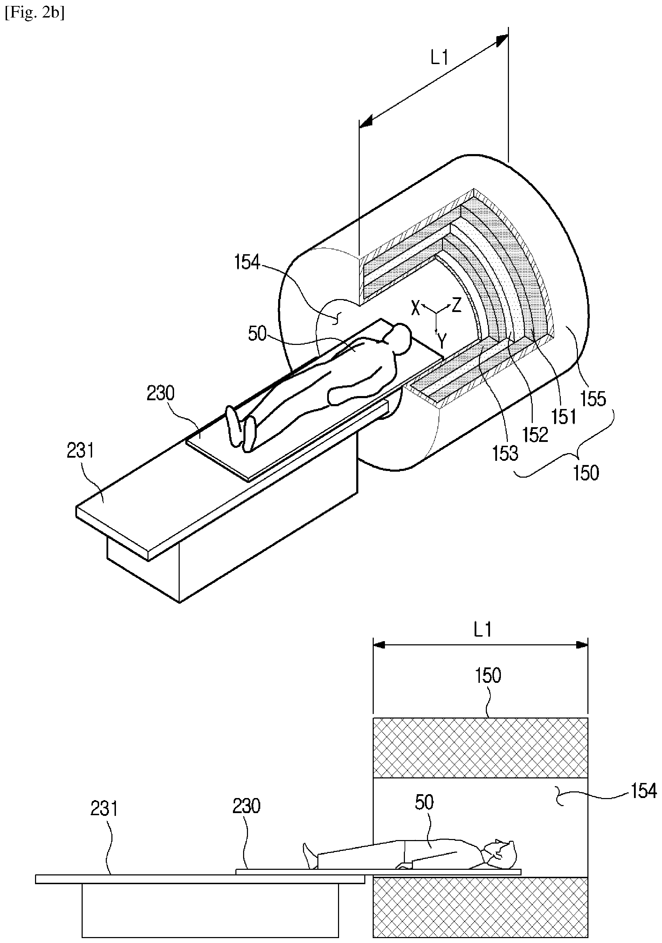

The gantry 150 may include a static field coil unit 151 for producing a static field in an internal space, a bore (154 of FIG. 2), a gradient coil unit 152 for producing a gradient field by making a gradient for a static field, and a Radio Frequency (RF) coil unit 153 for applying an RF pulse to the subject to excite an atomic nucleus and receiving an echo signal from the nucleus. The gantry 150 may also include a housing 155 (of FIG. 2) to prevent electromagnetic waves produced by the static field coil unit 151, gradient coil unit 152, or RF coil unit 153 from radiating to the outside.

The controller 120 may include a table controller 221 for controlling the movement of the table 230, a sequence controller 121 for planning a pulse sequence or controlling operation of the gantry 150 according to the pulse sequence, a content obtainer 160 for obtaining and providing content for the subject, and a screen controller 222 for controlling the image projector 270 and the screen unit 210.

The table controller 221, the sequence controller 121, and the screen controller 220 may be incorporated in a single processor or single device, or may be implemented separately in different processors or different devices.

The table controller 221 may control movement of the table 230 for carrying the subject into or out of the bore. In this regard, the whole subject may be carried into the bore, or only a part of the subject or a part targeted for examination may be carried into the bore.

The medical imaging apparatus 1 may further include a static field applier 131 for applying a static field current to the static field coil unit 151 to produce a static field, a gradient applier 132 for applying a gradient current to the gradient coil unit 152 to produce a gradient field, and an RF applier 133 for sending an RF signal to the RF coil unit 153. The sequence controller 121 may control a static field and a gradient field produced in the bore 154 and an RF applied to the subject by controlling the static field coil unit 151, the gradient coil unit 152, and the RF coil unit 153 with the static field applier 131, the gradient applier 132 and the RF applier, respectively.

The MR image obtainer 160 may be connected to the RF coil 153 for collecting magnetic resonance signals received by the RF coil unit 153. The MR image obtainer 160 may include a preamplifier for amplifying a magnetic resonance signal received by the RF coil 153, a phase detector for detecting a phase from a magnetic resonance signal sent from the preamplifier, an analog-to-digital (A/D) converter for converting an analog signal obtained by phase detection to a digital signal, and the like.

The MR image obtainer 160 may store magnetic resonance data digitally converted. The MR image obtainer 160 may include a k-space, and store the magnetic resonance data in the k-space by applying the two dimensional (2D) Fourier transform. Once k-space data is completed by filling the k-space with the magnetic resonance data, the MR image obtainer 160 may apply the Inverse Fast Fourier Transform (IFFT) and various image reconstruction schemes to the k-space data, to obtain a magnetic resonance image, i.e., an MR image.

The content obtainer 260 may obtain various content to be provided for the subject during the scanning process under the control of the screen controller 222. The content as used herein may include video, photos, scanning information (e.g., information regarding scanning time, scanning guide, scanned parts), etc., but is not limited thereto.

The content obtainer 260 may be connected to an external device or external server via a cable or by radio communication for receiving the content or storing the received content. For this, the content obtainer 260 may include a Universal Serial Bus (USB) port, and any communication module, such as a broadcast receiver module, a mobile communication module, a wireless Internet module, a short-range communication module, or the like.

The broadcast receiver module may include a terrestrial broadcast receiver module including an antenna, a demodulator, an equalizer, etc., for receiving terrestrial broadcasts, a Digital Multimedia Broadcasting (DMB) module for receiving and processing DMB broadcasts, or the like. The mobile communication module may perform communication by accessing a mobile communication network based on various mobile communication standards, such as 3rd Generation (3G), 3rd Generation Partnership Project (3GPP), Long Term Evolution (LTE), etc.

The wireless Internet module may perform communication over an external network according to a communication protocol, such as Wireless Local Area Network (WLAN), Wi-Fi, Wireless Broadband (Wibro), World Interoperability for Microwave Access (Wimax), High Speed Downlink Packet Access (HSDPA), etc.

The short-range communication module may perform communication with a nearby device according to a short-range communication scheme, such as Bluetooth, Radio Frequency Identification (RFID), Infrared Data Association (IrDA), Ultra Wideband (UWB), Zigbee, etc.

The content obtainer 260 may include a storage media for storing received content temporarily or non-temporarily. The storage media may be implemented in at least one of flash memory, hard disk, multimedia card micro type memory, card type memory (e.g., SD or XD memory), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, and optical disk. However, the storage media is not limited thereto, but may be implemented in any other form known in the art.

The aforementioned controller 120, MR image obtainer 160 or content obtainer 260 may be included in a workstation or a host device of the medical imaging apparatus 1. The entire controller 120 may be included in the workstation, or only a processor or some components of the controller 120 may be included in the workstation. Likewise, the entire MR image obtainer 160 may be included in the workstation, or only a processor or some components of the MR image obtainer 160 may be included in the workstation, and the entire content obtainer 260 may be included in the workstation, or only a processor or some components of the content obtainer 260 may be included in the workstation.

Although the controller 120, the MR image obtainer 160, and the content obtainer 260 are shown to be separated from each other, at least two of them may be incorporated in a single processor or a single device.

The image projector 270 may include a light source and a projecting lens for projecting an image included in the content to the screen unit 210. The image projector 270 may be mounted on the table 230 or on a support (231 of FIG. 2) for supporting the table 230.

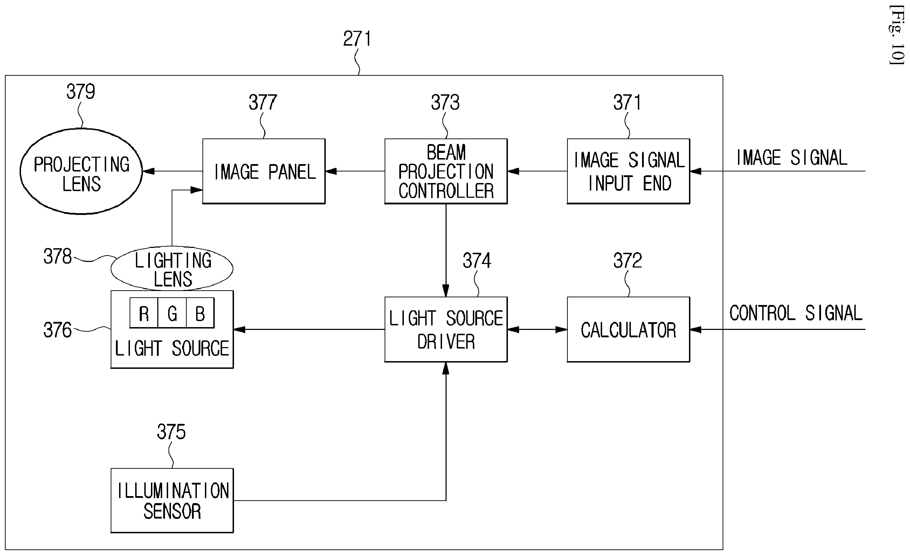

The screen unit 210 may include a screen that reflects off the image projected from the image projector 270. The screen unit 210 may be implemented in the form of a film or a panel. The screen itself may be implemented in the form of a panel, or a face (e.g., the front face or the rear face) of the screen may be combined with a panel. The panel may be formed of a flexible substance. The screen unit 210 may be flat or curved. The screen unit 210 may be arranged on the gantry 150 or on the ceiling 150 (C of FIG. 10) of the examination room for allowing the subject to enjoy the reflected image. The image projector 270 and the screen unit 210 will be described later in more detail.

The user interface unit 110 may include an input unit 111 and a display unit 112 for providing a user interface to input various information regarding scanning or to control various devices. For example, the user may set conditions per part for examination or input an instruction to move the table 230, an instruction to start scanning, an instruction to select content to be projected, an instruction to start or stop content projection, etc., through the user interface unit 110. The user interface unit 110 may also receive an instruction regarding a scanning sequence from the user and accordingly, generate a pulse sequence. In addition, the user check MR images obtained in the scanning process through the user interface unit 110.

The input unit 111 may include many different buttons or switches, a pedal, a keyboard, a mouse, a track ball, various levers, a handle, a stick, or some hardware input devices for the user input. The input unit 111 may also include a Graphical User Interface (GUI), i.e., a software input device, such as a touch pad for the user input. The touch pad may be implemented with a Touch Screen Panel (TSP), thus forming a interlayer structure with the display unit 112.

The display unit 112 may include a Cathode Ray Tube (CRT), a Digital Light Processing (DLP) panel, a Plasma Display Panel (PDP), a Liquid Crystal Display (LCD) panel, an Electro Luminescence (EL) panel, an Electrophoretic Display (EPD) panel, an Electrochromic Display (ECD) panel, a Light Emitting Diode (LED) panel, an Organic Light Emitting Diode (OLED) panel, etc., but is not limited thereto.

As noted above, the display unit 112 may also be used as an input device in addition to the display device if implemented with the TSP to form with the interlayer structure with the touch pad.

The user interface unit 110 may be included in the workstation or host device of the medical imaging apparatus 1, but is not limited thereto, and the input unit 111 and display unit 112 of the user interface unit 110 may be separately located.

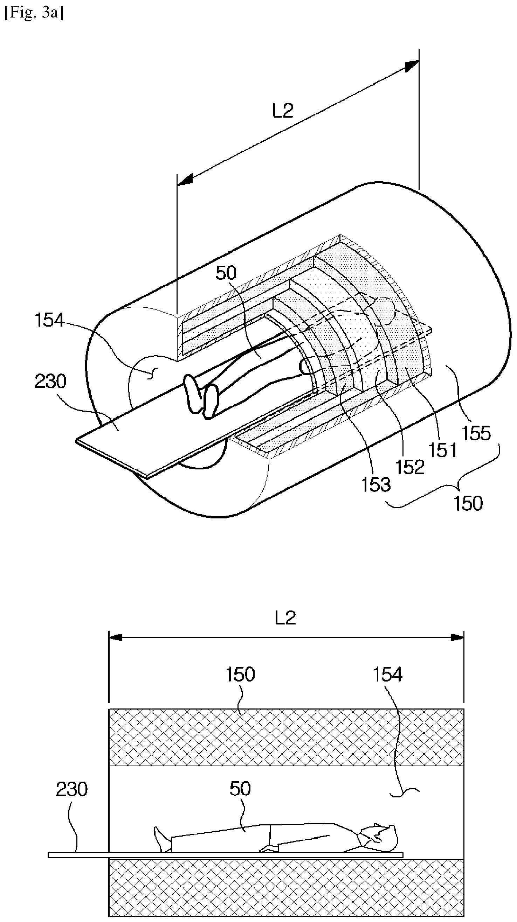

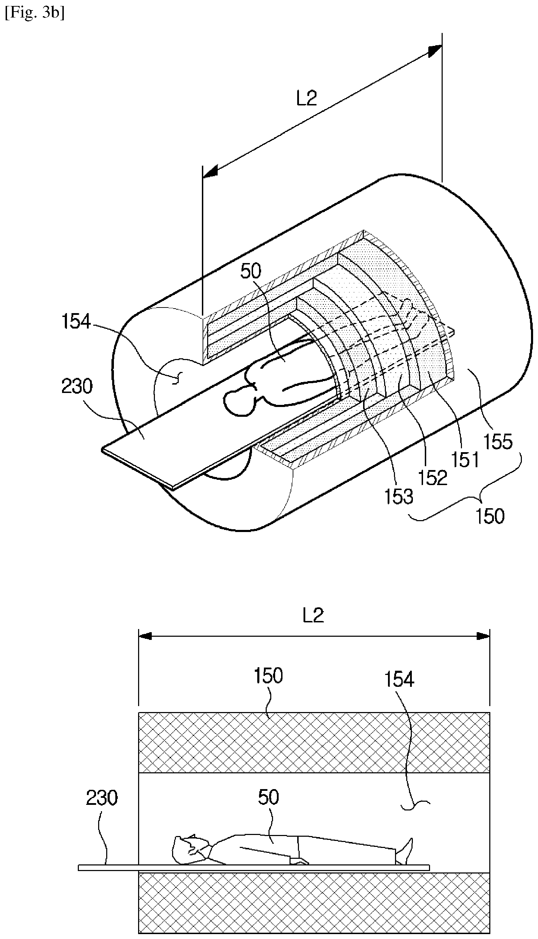

FIGS. 2A and 2B show the exterior of a medical imaging apparatus, according to an embodiment of the present disclosure, and FIGS. 3A and 3B show the exterior of a medical imaging apparatus, according to another embodiment of the present disclosure. In these embodiments, the image projector 270 and the screen unit 210 are assumed to be hidden.

Referring to FIGS. 2A, 2B, 3A, and 3B, the gantry 150 may sequentially include a static field coil unit 151, a gradient coil unit 152, and an RF coil 153, and may have a shape of a hollow cylinder. The internal space of gantry 150 may be called a bore 154 or cavity.

The table 230 may serve to carry a subject 50 lying on the table 230 into the bore 154. If the user inputs an instruction regarding a location of the table 230 by manipulating the input unit 111, the table controller 221 may generate a control signal corresponding to the user instruction to move the table 230 to the location desired by the user in the longitudinal direction of the support 231, i.e., along the Z-axis.

The static field coil unit 151 may include a coil for producing a static field in the bore 154, the coil also called a main magnet that may be implemented with a superconducting magnet. In this case, the static field coil unit 151 may include a superconducting coil.

The static field coil unit 151 may be arranged in a way that the coil is wound on the circumference of the bore 154, and a static field with certain strength may be produced when a current is applied from the static field applier 131 to the static field coil unit 151. The direction of the static field corresponds to the longitudinal axis of the gantry 150.

Once the static field is produced in the bore 154, nuclei of atoms, especially of hydrogen atoms are aligned in the direction of the static field, and precess about the direction of the static field.

The nuclei's speed of precession may be represented by a precession frequency, called a Larmor frequency, which may be expressed in the following equation 1:

$$\omega = \gamma B0 \ (1)$$

$\omega$ represents a Larmor frequency, $\gamma$ represents a proportional factor, and B0 represents a strength of an external magnetic flux. The proportional factor may vary by type of nucleus, and a Tesla (T) or Gauss (G) is a unit of the strength of the external magnetic flux and a hertz (Hz) is a unit of a precession frequency.

For example, a proton of hydrogen has a procession frequency of 42.58MHz in IT of external magnetic flux, and since hydrogen accounts for the biggest percentage among atoms that make up a living body, the precession of protons of hydrogen is mainly used to obtain a magnetic resonance signal in the MRI.

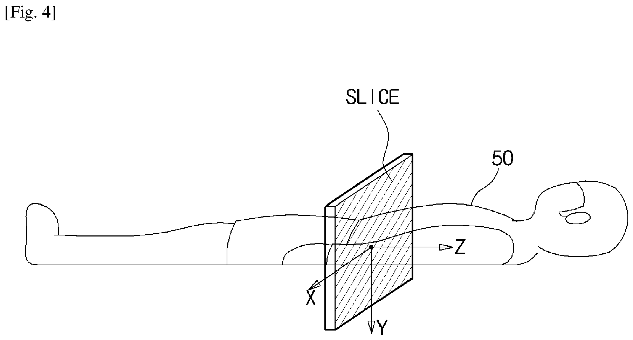

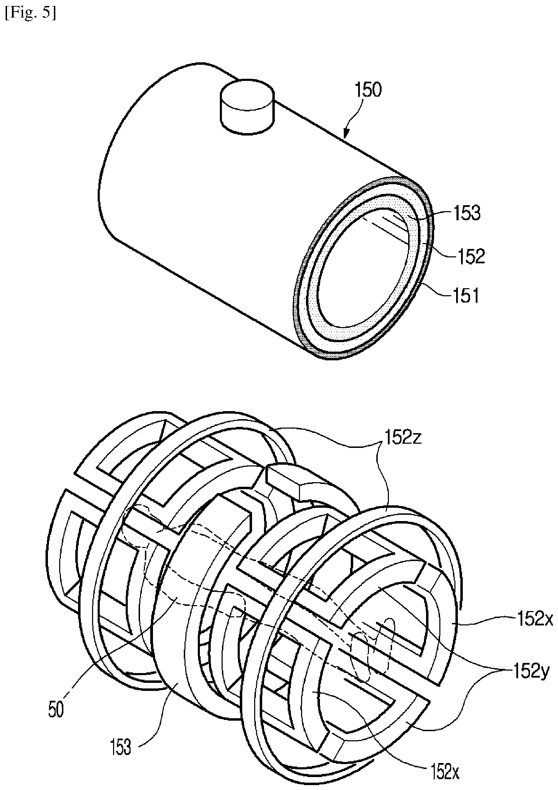

FIG. 4 shows a space where a subject is lying down, which is classified into X, Y, and Z axes, and FIG. 5 shows structures of a gantry and a gradient coil unit.

As shown in FIG. 4, under assumption that the longitudinal axis of the subject 50 lies in parallel with the longitudinal axis of the gantry 150, an axis that corresponds to the longitudinal axis of the subject from head to toe, i.e., an axis in parallel with the direction of the static field may be determined as the Z-axis, an axis that corresponds to the lateral direction of the subject (also called patient 50) may be determined as the X-axis, and an axis that corresponds to the vertical direction of the space may be determined as the Y-axis.

When the longitudinal axis of the subject 50 corresponds to the direction of the static field, a tomogram of a cross section of the patient 50 may be obtained. To obtain the tomogram, a slice with a certain thickness may be selected.

To obtain three dimensional (3D) spatial information regarding the magnetic resonance signal, gradient fields for all the X, Y, and Z axes are required. Accordingly, the gradient coil unit 152 may include three pairs of gradient coils, which correspond to X, Y, and Z axes, respectively.

As shown in FIG. 5, gradient coils for Z-axis 152z may generally include a pair of ring type coils, and gradient coils for Y-axis 152y may be located above and below the subject 50. Gradient coils for X-axis 152x may be located on the left and right sides of the subject 50.

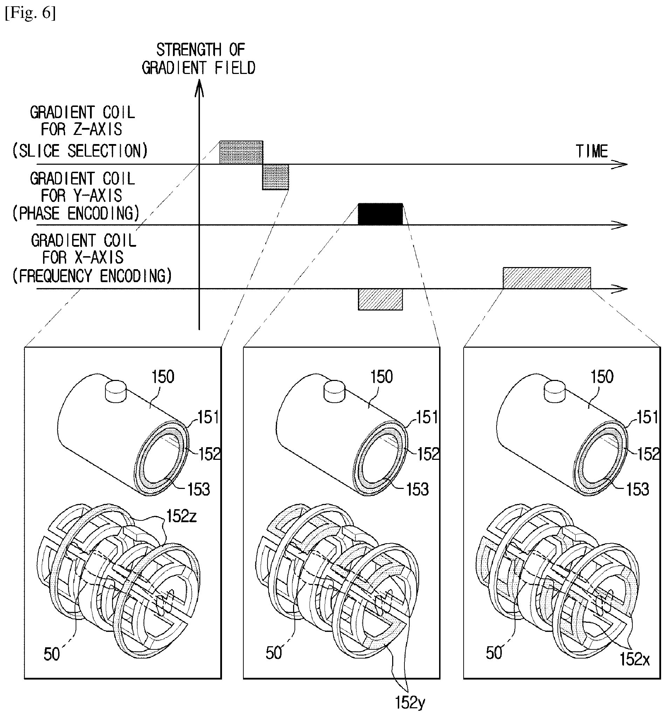

FIG. 6 shows a pulse sequence related to respective operation of gradient coils that constitute the gradient coil unit 152.

When direct currents with opposite polarities flow through two gradient coils for Z-axis 152z in the opposite directions, a change in magnetic field occurs in the Z-axis, thus producing a gradient field.

While the gradient field is produced by the currents flowing through the gradient coils for Z-axis 152z for a predetermined time, the resonance frequency is changed by the strength of the gradient field. If the RF coil unit 153 produces an RF for a particular location, only protons in a cross section corresponding to the particular location produce resonance. Accordingly, the gradient coils for Z-axis 152z are used for slice selection. As the gradient of the gradient field produced in the Z-axis increases, a thinner slice may be selected.

If a slice is selected based on the gradient field produced by the gradient coils for Z-axis, all spins that constitute the slice have the same frequency and the same phase, which makes it impossible to tell a spin from another.

At this time, if a gradient field is produced by the gradient coils for Y-axis 152y in the Y-axis, the gradient field may cause phase shift to make rows of the slice have different phases.

In other words, once the gradient field is produced in the Y-axis, spins of rows to which strong magnetic fields are applied undergo a phase change into higher frequencies, while spins of rows to which weak magnetic fields are applied undergo a phase change into lower frequencies. If the gradient field in the Y-axis disappears, respective rows of a selected slice are phase-shifted to different phases, which enables the rows to be distinguished. The gradient field produced by the gradient coils for Y-axis 152y may be used in phase encoding.

As such, a slice is selected according to a gradient field produced by the gradient coils for Z-axis 152z, and rows constituting the selected slice may be distinguished by different phases caused by a gradient field produced by the gradient coils for Y-axis 152y. However, the spins constituting a row may not be distinguished because they all have the same frequency and the same phase.

In this regard, if a gradient field in X-axis is produced by the gradient coils for X-axis 152x, the gradient field in the X-axis may cause the spins constituting each row to have different frequencies, thus enabling the respective spins to be distinguished. As such, the gradient field produced by the gradient coils for X-axis 152x may be used in frequency encoding.

As discussed above, the gradient fields produced by the gradient coils for Z-, Y-, and X-axes may enable spatial encoding of spatial positions of respective spins through slice selection, phase encoding, and frequency encoding.

The gradient coil unit 152 may be coupled with the gradient applier 132, which applies gradient waves, i.e., current pulses to the gradient coil unit 152 according to a control signal sent from the sequence controller 121 to produce a gradient field. Accordingly, the gradient applier 132 may also be called a gradient power source, including three diving circuits for the three pairs of gradient coils 152x, 152y, and 152z that constitute the gradient coil unit 152.

The gradient applier 132 may include a pulse generator for generating gradient waves (current pulses) in a pulse sequence planned by the sequence controller 122, and a gradient amplifier for amplifying the gradient waves and deliver the amplified gradient waves to the gradient coil unit 152.

As discussed earlier, the atomic nuclei aligned by an external magnetic field presses at the Larmor frequency, and the vector sum of magnetization of the multiple atomic nuclei may be represented by net magnetization M.

It is not possible to measure a Z-axis component of average magnetization but MXY. To obtain a magnetic resonance signal, the average magnetization is forced to be on the XY plane by excitation of the nuclei. For excitation of the nuclei, RF pulses tuned to the Larmor frequency of the nuclei need to be applied.

The RF applier 133 may be coupled with the RF coil unit 153, to which the RF pulse tuned to the Larmor frequency is applied. The RF applier 133 may include a modulation circuit for modulating a high frequency signal to a pulse signal, and an RF power amplifier for amplifying the pulse signal.

The RF coil unit 153 may include a transmit coil for transmitting RF pulses and a receive coil for receiving electromagnetic waves irradiated by excited atomic nuclei, i.e., a magnetic resonance signal. Alternatively, without having the transmit and receive coils separately, a coil capable of both transmission and reception, such as a head coil may be used.

As discussed earlier, the magnetic resonance signal received by the RF coil unit 153 may be collected by the MR image obtainer 160 for obtaining an MR image.

Turning back to FIGS. 2A, 2B, 3A, and 3B, the gantry 150 may be formed to have a length L1 only to cover a part of the table 230 (or the subject 50) as shown in FIGS. 2A and 2B, or a length L2 to cover the entire table 230 (or the subject 50) as shown in FIGS. 3A and 3B. The gantry 150 formed to have the length only to cover a part of the table 230 (or the subject 50) is called a first gantry, and the gantry 150 formed to have the length to cover the entire table 230 (or the subject 50) is called a second gantry.

If the gantry 150 corresponds to the first gantry, a feet-first direction and a head-first direction may be defined depending on a part for examination. Specifically, if a part under the waist of the subject 50 is to be examined, the subject 50 would be lying on the table 230 with his/her feet located toward the bore 154 and then the part under the waist would be carried into the bore 154 by movement of the table 230, which may be defined as the feet-first direction. If a part above the waist of the subject 50 is to be examined, the subject 50 would be lying on the table 230 with his/her head located toward the bore 154 and then the part above the waist would be carried into the bore 154 by movement of the table 230, which may be defined as the head-first direction.

On the other hand, if the gantry 150 corresponds to the second gantry, as shown in FIGS. 3A and 3B, regardless of where the head of the subject 50 is located, the entire table 30 or subject 50 may be located inside the bore 154 by movement of the table 230. Even in this case, it is possible to put only a part to be examined (e.g., a part under the waist) into the bore 154 in order to reduce the magnetic field or electromagnetic waves affecting the subject 50.

In other words, if the gantry 150 corresponds to the first gantry or even to the second gantry, the head of the subject 50 may occasionally be outside of the bore 154, in which case the image projector 270 may project various content to relieve boredom or inconvenience of the patient during the scanning process.

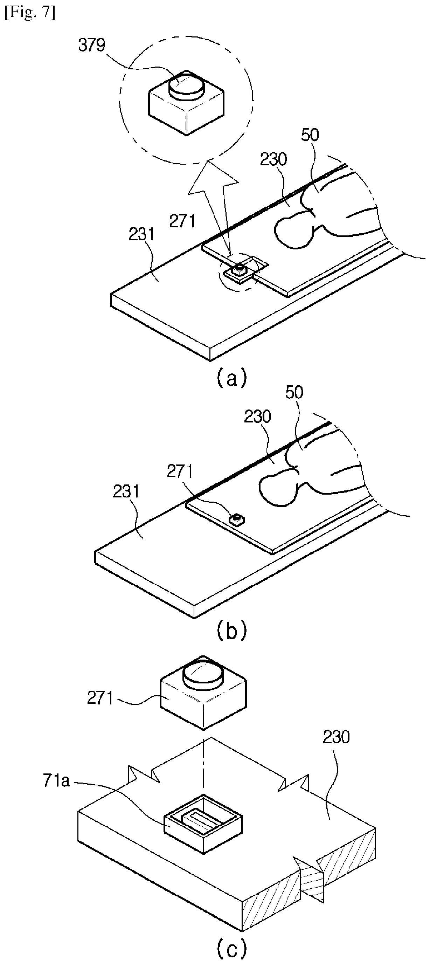

FIG. 7 shows a schematic arrangement of an image projector, according to an embodiment of the present disclosure.

Referring to FIG. 7, the image projector 270 may be implemented as a beam projector 271 that includes a projecting lens 379 arranged on the top of the beam projector 271. The beam projector 271 may be mounted on the table 230. The beam projector 271 may be mounted on the side face of the table 230, as shown in (a) of FIG. 7, or on the end of the top face of the table 230, as shown in (b) of FIG. 7, in order for the projecting lens 379 not to be hidden by a subject when the subject lies on the table 230. The beam projector 271 may be mounted in the way that protrudes from the table 230 or sinks in the table 230, or in the way that is pushed into or pulled out from a concave space of the table 230. Alternatively, as shown in (c) of FIG. 7, a detachable module 71a is mounted on the table 230, and the beam projector 271 may be detachably attached to the detachable module 71a. Where and how the beam projector 271 is mounted on the table 230, or what form it takes when the beam projector 271 is mounted are not limited to what are described above.

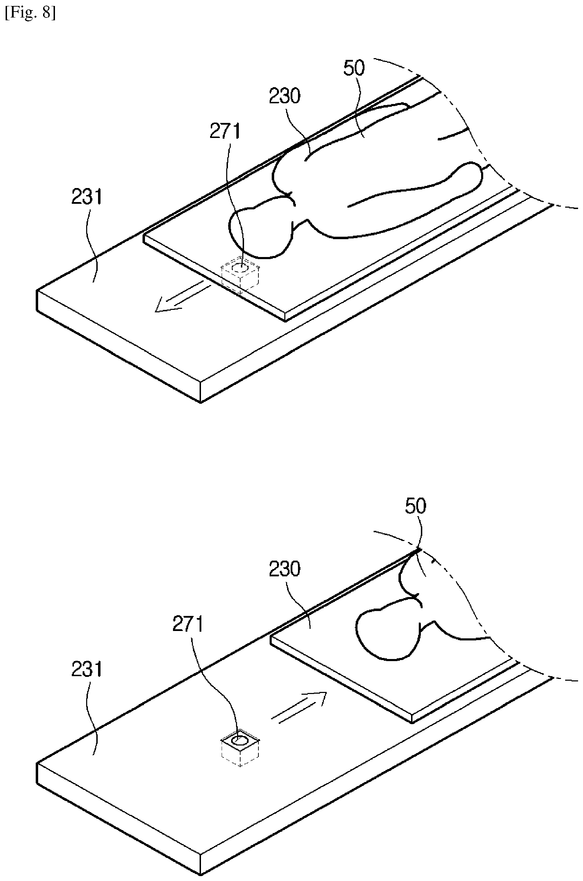

FIG. 8 shows an image projector mounted on a supporter, according to an embodiment of the present disclosure.

Referring to FIG. 8, the image projector 270 implemented as the beam projector 271 may be mounted on a supporter 231. The beam projector 271 may be mounted in the way that sinks in the supporter 231 or in the way that is pushed into or pulled out from a concave space of the supporter 231. The beam projector 271 may be mounted in the way that is closed by the table 230 but open by movement of the table 230 toward the bore 154.

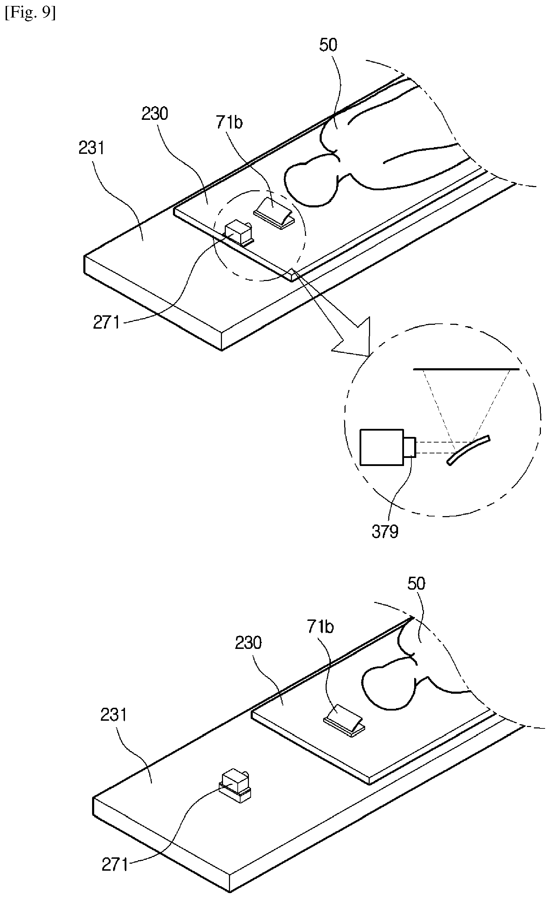

FIG. 9 shows a schematic arrangement of an image projector, according to another embodiment of the present disclosure.

Referring to FIG. 9, the image projector 270 may include a beam projector 271 and a reflecting mirror 71b. A projecting lens 379 of the beam projector 271 may be arranged to face the reflecting mirror 71b, so that an image projected from the beam projector 271 may be reflected upward by the reflecting mirror 71b. The reflecting mirror 71b may be formed to have an adjustable slope, and an angle of reflection of the projected image may be adjusted based on the slope of the reflecting mirror 71b. As shown in the upper diagram of FIG. 9, the beam projector 271 and the reflecting mirror 71b may all be mounted on the table 230. Alternatively, as shown in the lower diagram of FIG. 9, the beam projector 271 may be mounted on the supporter 231 and the reflecting mirror 71b may be mounted on the table 230. At least one of the beam projector 271 and the reflecting mirror 71b may be detachably mounted.

FIG. 10 is a block diagram of a beam projector, according to an embodiment of the present disclosure.

Referring to FIG. 10, the beam projector 271 may include an image signal input end 371, a calculator 372, a beam projection controller 373, a light source driver 374, an illumination sensor 375, a light source 376, a lighting lens 378, an image panel 377, and a projecting lens 379.

If the beam projector 271 receives content from the aforementioned content obtainer 260, an image signal contained in the content is input to the image signal input end 371 and then delivered to the beam projection controller 373. The beam projection controller 373 may convert the image signal to an image signal for beam projection, and send the resultant image signal to the image panel 377. The image panel 377 may be an ordinary image panel known to the public, such as a transparent LCD panel, a transparent LED panel, a transparent OLED panel, or reflective Digital Micromirror Device (DMD) panel. The beam projection controller 373 may also send a light source driver signal for the image signal for beam projection to the light source driver 374.

The calculator 372 may receive a control signal from the aforementioned screen controller 222, and control the image signal input end 371 or the light source driver 374 according to the control signal.

The image panel 377 may display an image based on the image signal for beam projection. When the light source 376 is driven by the light source driver signal, light beams illuminated through the lighting lens 378 may be modulated by an image displayed on the image panel 377 and projected through the projecting lens 379 while transmitting or reflecting off the image panel 377. The projecting lens 379 may passively or actively adjust a focal point for a projected image.

The illumination sensor 375 may detect illumination of the inside of the examination room. Information about the illumination detected by the illumination sensor 375 may be delivered to the light source driver 374 to adjust brightness in driving the light source 376. The illumination detected by the illumination sensor 275 may vary by position of the screen unit 210. Accordingly, brightness of the light source 167 may be controlled for the position of the screen unit 210. In other words, the brightness of an image to be displayed on the screen unit 210 may be properly kept by varying an amount of light for an image projected from the beam projector 271 based on the position of the focused image.

Since images for beam projection have various resolutions and various screen sizes, the beam projection controller 273 may perform signal scaling processing on the input image signal to suit a format (of e.g., resolution, screen size) for beam projection. Such a format may be set and stored in advance, or may be set through the input unit 111. Once content for projection is selected by the user's manipulation through the input unit 111, the screen controller 222 may send a request for an image output change for the content for projection to the beam projector 271, and the beam projection controller 373 may perform the image output change to suit the set format. Such a scaling function may naturally be included in the screen controller 222.

The beam projector 271 may generate heat e.g., while driving the light source 376, and the heat may critically influence the subject. Accordingly, the beam projector 271 may further include a heatproof member (not shown). The heatproof member may employ a heat sink, a cooling fan, etc., for example. The beam projector 271 may have an electromagnetic shield for not influencing or protecting against an electromagnetic field of the bore 154, or may have a circuit to minimize influence of a high magnetic field of the bore 154.

Such configuration of the beam projector 271 is not limited thereto; some components may be omitted or added, or multiple components may be implemented in a combined form, or a component may be implemented to be divided into multiple components.

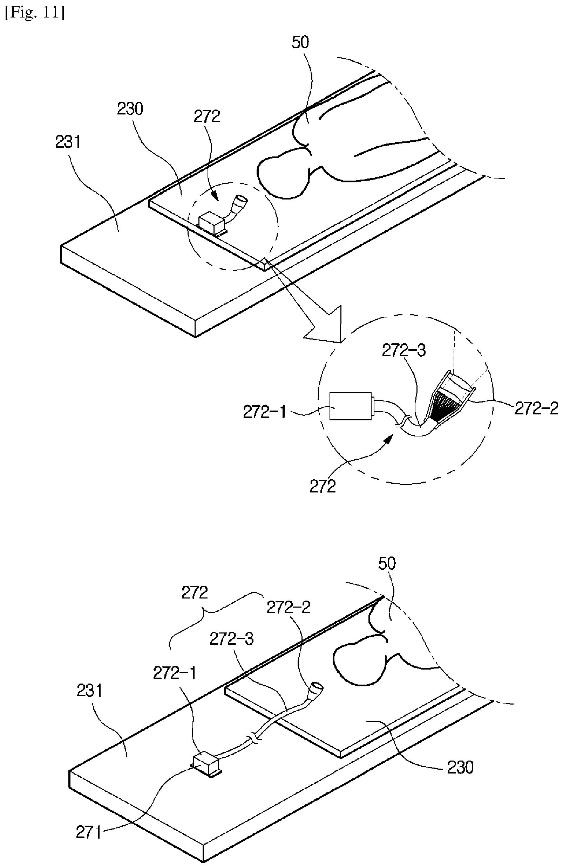

FIG. 11 shows a schematic arrangement of an image projector, according to an embodiment of the present disclosure.

Referring to FIG. 11, the image projector 270 may include a projecting lens unit 271-1 and a light source unit 272-2, which may be interconnected via an optic fiber cable 272-3.

The light source unit 272-2 may receive content from the content obtainer 260, and produce a light beam according to an image signal of the content. The light source unit 272-2 may deliver the light beam containing an image to the projecting lens unit 271-1 via the optic fiber cable 272-3. The optic fiber cable 272-3 may include multiple optic fibers, which may be installed at an output end of the light source unit 272-2 and the incident end of the projecting lens unit 271-1 in the same array. Accordingly, the multiple optic fibers of the optic fiber cable 272-3 may deliver the light beam produced by the light source unit 272-2 to the projecting lens unit 271-1 while keeping the image intact. The light beam delivered to the projecting lens unit 271-1 with the image may be projected onto the screen unit 210 through the projecting lens unit 271-1.

As shown in the upper diagram of FIG. 11, the projecting lens unit 271-1 and the light source unit 272-2 may all be mounted on the table 230. Alternatively, as shown in the lower diagram of FIG. 11, light source unit 272-2 may be mounted on the supporter 231 and the projecting lens unit 271-1 may be mounted on the table 230. In the latter case, the optic fiber cable 272-3 may be flexible, and may hold the optical connection between the projecting lens unit 271-1 and the light source unit 272-2 even if the distance between them is changed by movement of the table 230.

How the image projector 270 is implemented has been described above in connection with FIGS. 8 to 11, but it is not limited thereto, and any device that is able to project an image contained in the content may be employed as the image projector 270. The image projector 270 may be implemented in singular form or plural form. Furthermore, the image projector 270 may be rotationally implemented, which will be described later.

The screen unit 210 may display an image projected by the image projector 270, i.e., an image contained in the content in a way of reflection, enabling the subject 50 to see the image during the scanning process. The screen unit 210 may implement out bore display. The out bore display herein used may refer to displaying an image outside the bore, and in bore display, by contrast, may be defined as displaying an image inside the bore. The screen unit 210 may be implemented in singular or plural form, and may be mounted on the gantry 150 or the ceiling C. The screen unit 210 may be mounted in at least one of push/pull method, rotation method, and fixed method.

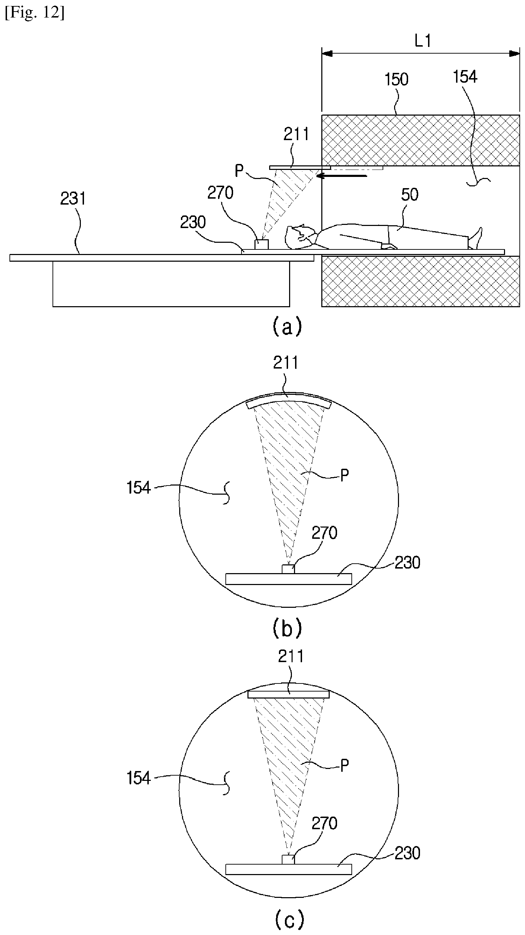

FIG. 12 is a diagram for explaining a screen unit, according to an embodiment of the present disclosure.

Referring to FIG. 12, a screen unit 211 may be mounted on the gantry 150 in a push/ pull method, and such a screen unit may be referred to as a push/pull screen unit.

The push/pull screen unit 211 may be implemented to be pushed into the gantry 150 or pulled out of the gantry 150. For example, a guide rail or a guide groove may be installed on the gantry 150, and the push/pull screen unit 211 may be installed to slide along the guide rail. The push/pull screen unit 211 may be pushed into the gantry 150 or pulled out of the gantry 150 while sliding along the guide rail. The push/pull screen unit 211 may be implemented to have a curved form to correspond to the shape of the gantry 150, as shown in (b) of FIG. 12, or to have a flat form, as shown in (c) of FIG. 12.

In the meantime, if the gantry 150 is the first gantry, the medical imaging apparatus 1 may scan the subject in the feet-first direction or head-first direction depending on which part is to be examined, as described above.

As shown in (a) of FIG. 12, in a case the subject 50 is scanned in the first gantry 150 in the feet-first direction, the push/pull screen unit 211 may be pulled out of the gantry 150 and the image projector 270 may project a content image onto the push/pull screen unit 211 that is pulled out of the gantry 150. On the other hand, in a case the subject 50 is scanned in the head-first direction, the push/pull screen unit 211 may be pushed into the gantry 150 and the image projector 270 may stop projecting the content image.

In other words, the push/pull screen unit 211 mounted on the first gantry 150 may be pulled out to implement the out bore display when the subject 50 is scanned in the feet-first direction, and pushed in when the subject 50 is scanned in the head-first direction, thereby implementing no out bore display nor in bore display.

Pushing or pulling the push/pull screen unit 211 may be performed in response to a user instruction input through the input unit 111. The image projector 270 may actively or passively project or stop projecting a content image in response to whether the push/ pull screen unit 211 is pushed in or pulled out.

Active projection or stop projection of a content image by the image projector 270 may be performed in response to whether the screen controller 222 detects the push/ pull screen unit 211 being pushed or pulled. Specifically, when the push/pull screen unit 211 is pulled out, the screen controller 222 may detect this and generate a control signal to control the image projector 270 to project a content image. When the push/ pull screen unit 211 is pushed in, the screen controller 222 may detect this and generate a control signal to control the image projector 270 not to project or to stop projecting a content image.

Passive projection or stop projection of a content image by the image projector 270 may be performed by an instruction of the user through the input unit 111. Specifically, the user may input an instruction to start image projection through the input unit 111 when the user is aware that the push/pull screen unit 211 has been pulled out, and then the image projector 270 may start projecting a content image in response to the user's instruction. Also, in response to the user's instruction to stop projection after the user is aware that the push/pull screen unit 211 has been pushed in, the image projector 270 may not project or may stop projecting a content image.

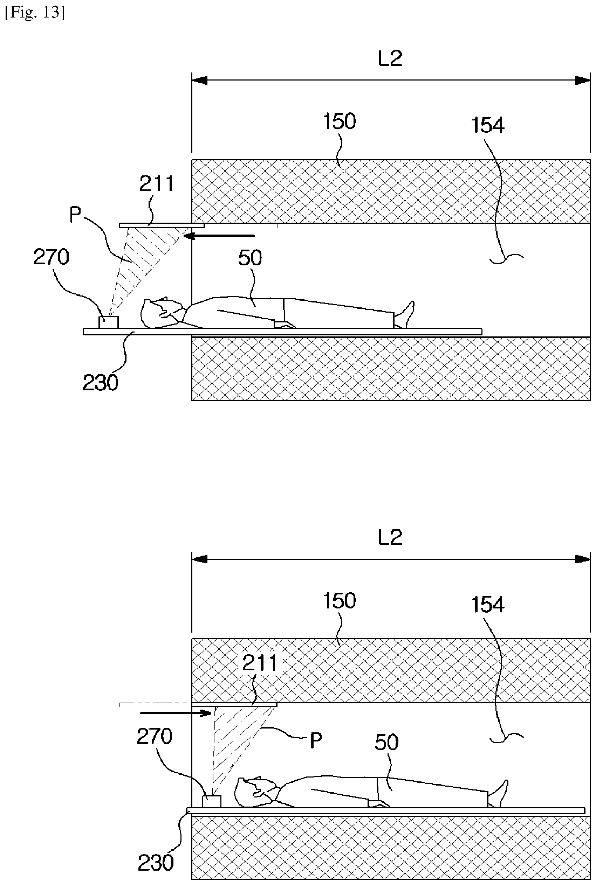

FIG. 13 shows an occasion where a push/pull screen unit is mounted on the second gantry.

Referring to FIG. 13, the push/pull screen unit 211 may be mounted on the second gantry. As shown in the upper diagram of FIG. 13, in a case the head of the subject 50 is located outside the bore, the push/pull screen unit 211 may be pulled out of the gantry 150 and the image projector 270 may project a content image onto the push/pull screen unit 211 that is pulled out of the gantry 150. As shown in the lower diagram of FIG. 13, in a case the head of the subject 50 is located inside the bore, the push/pull screen unit 211 may be pushed into the gantry 150. In this case, however, the image projector 270 may stop projecting the content image or keep on projecting the content image, depending on the system settings or the user's instruction through the input unit 111.

In other words, the push/pull screen unit 211 mounted on the second gantry 150 may implement the out bore display by being pulled out when the head of the subject 50 is located outside the bore. When the head of the subject 50 is located inside the bore, the push/pull screen unit 211 may be pushed in, thus not implementing the out bore display but implementing the in bore display depending on a setting. The push/pull screen unit 211 mounted on the second gantry 150 may perform the out bore display function or the in bore display function, depending on whether the push/pull screen unit 211 is pulled out or pushed in.

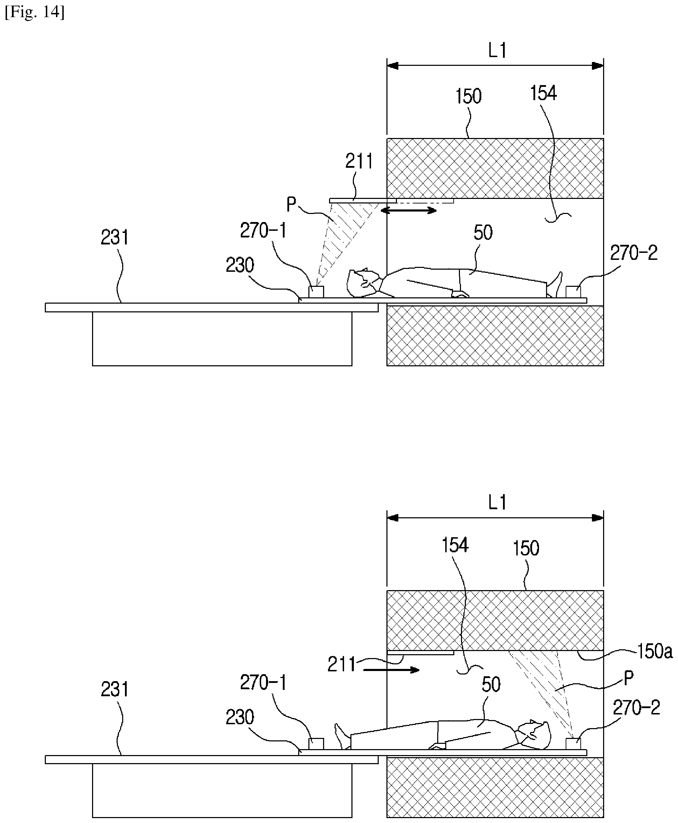

As discussed above, the image projector 270 may be implemented in plural form. FIG. 14 shows a medical imaging apparatus having the first gantry on which the push/ pull screen unit is mounted, and a plurality of image projectors.

Referring to FIG. 14, the medical imaging apparatus 1 may include the first gantry on which the push/pull screen unit 211 is mounted, and two image projectors 270-1, 270-2. The two image projectors 270-1, 270-2 may be mounted on either end of the table 230, the one mounted on the left end called a first image projector 270-1 and the other one mounted on the right end called a second image projector 270-2.

As shown in the upper diagram of FIG. 14, in the case the subject 50 is scanned in the first gantry 150 in the feet-first direction, the push/pull screen unit 211 may be pulled out of the gantry 150 and the first image projector 270-1 may project a content image onto the push/pull screen unit 211 which is pulled out of the gantry 150. On the other hand, as shown in the lower diagram of FIG. 14, in the case the subject 50 is scanned in the head-first direction, the push/pull screen unit 211 may be pushed into the gantry 150 and the first image projector 270-1 may stop projecting the content image. In this case, the second image projector 270-2 may project a content image, which is reflected off an inner wall 150a of the gantry 150.

In other words, the push/pull screen unit 211 mounted on the first gantry 150 may be pulled out to implement the out bore display when the subject 50 is scanned in the feet-first direction, and pushed in when the subject 50 is scanned in the head-first direction, thereby implementing no out bore display nor in bore display. In the latter case, however, the in bore display function may be performed when the inner wall 150a of the gantry reflects the projected image from the second image projector 270-2. In other words, with the multiple image projectors 270, both the out bore display and the in bore display may be implemented.

Similar to the image projector 270 implemented in plural form as shown in FIG. 14, the screen unit 210 may also be implemented in plural form. For example, the screen unit 210 may be implemented with a plurality of push/pull screen units 211.

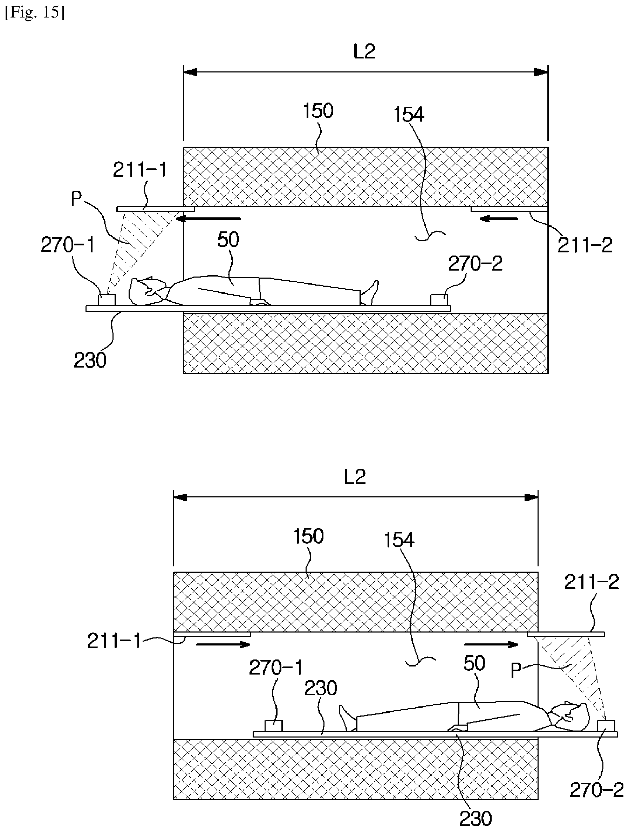

FIG. 15 shows a medical imaging apparatus having the second gantry on which a plurality of push/pull screen units are mounted, and a plurality of image projectors.

Referring to FIG. 15, the medical imaging apparatus 1 may include the second gantry on which push/pull screen units are mounted, and first and second image projectors 270-1 and 270-2 mounted on either end of the table 230. The push/pull screen units 211-1, 211-2 may be mounted on either side of the gantry 150, the one mounted on the left side called a first push/pull screen unit 211-1 and the other one mounted on the right side called a second push/pull screen unit 211-2.

As shown in the upper diagram of FIG. 15, in a case the head of the subject 50 is located outside the bore on the left, the first push/pull screen unit 211-1 may be pulled out of the gantry 150 and the first image projector 270-1 may project a content image onto the first push/pull screen unit 211-1 which is pulled out of the gantry 150. In this case, the second push/pull screen unit 211-2 located on the right may be pushed into the gantry 150.

As shown in the lower diagram of FIG. 15, in a case the head of the subject 50 is located outside the bore on the right, the second push/pull screen unit 211-2 may be pulled out of the gantry 150 and the second image projector 270-2 may project a content image onto the second push/pull screen unit 211-2 which is pulled out of the gantry 150. In this case, the first push/pull screen unit 211-1 located on the left may be pushed into the gantry 150.

In other words, the first push/pull screen unit 211-1 mounted on the second gantry 150 may be pulled out to implement the out bore display when the head of the subject 50 is located outside the bore on the left, and the second push/pull screen unit 211-2 may be pulled out to implement the out bore display when the head of the subject 50 is located outside of the bore on the right. In the case the head of the subject 50 is located inside the bore, the plurality of push/pull screen units 211-1, 211-2 may be pushed in, and one of the push/pull screen units 211-1, 211-2 (i.e., the first push/pull screen unit 211-1 or the second push/pull screen unit 211-2) may perform the in bore display function depending on the direction of the head of the subject 50.

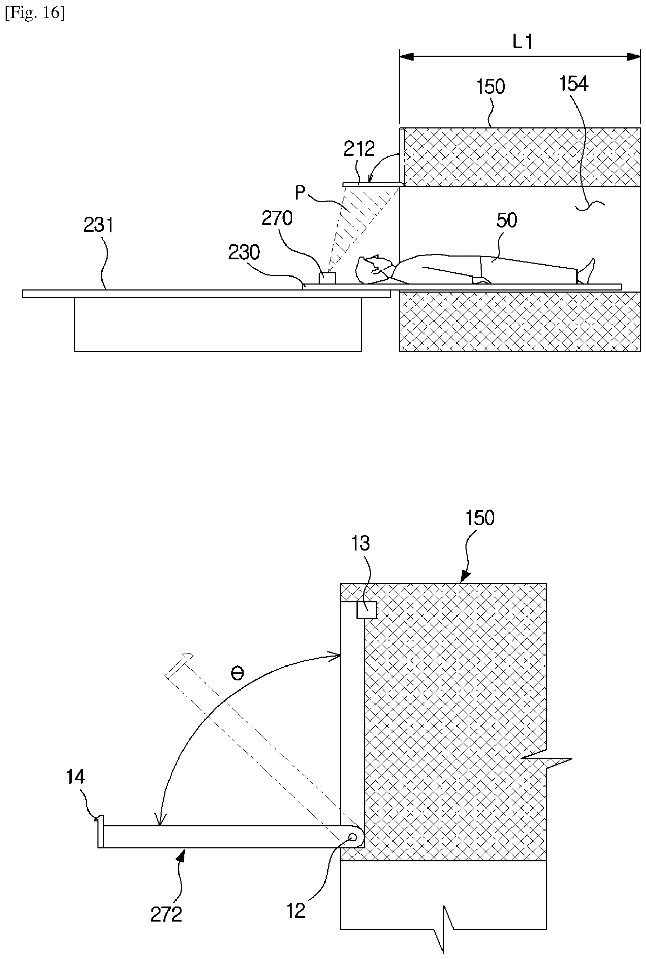

FIG. 16 is a diagram for explaining a screen unit, according to another embodiment of the present disclosure.

Referring to FIG. 16, a screen unit 212 may be rotationally mounted on the outside of the gantry 150, which will now be referred to as a rotational screen unit.

The rotational screen unit 212 may be combined with the gantry 150 on one end, and arranged to have the other end rotated upward or downward about the one end as the rotation axis. For example, a hinge device 12 may be installed on the one end of the rotational screen unit 212 to rotationally combine the rotational screen unit 212 with the gantry 150.

One face R of the rotational screen unit 212 may keep in contact with the gantry 150, but separated from the gantry 150 when rotated. To keep the rotational screen unit 212 in contact with the gantry 150, a hook or magnet may be mounted on the one face R or on the other end of the rotational screen unit 212. For example, a sill 13 may be formed in the gantry 150, and a hook 14 may be formed at a position corresponding to the sill 13 on the other end of the rotational screen unit 212. The one face R of the rotational screen unit 212 may then be combined with the gantry 150 with the hook 14 stuck to the sill 13. Specifically, the rotational screen unit 212 may be positioned at 0 rotation degree, which will be said that 'the rotational screen unit 212 is closed'.

The rotational screen unit 212 may be positioned at a maximum rotation degree as well. The rotational screen unit 212 may be positioned at a certain rotation degree θ to face the image projector 270, and the certain rotation degree θ may be equal to the maximum rotation degree. The certain rotation degree may be set and stored in advance. The rotational screen unit 212 being rotated by the certain rotation degree θ will be said that 'the rotational screen unit 212 is open'.

As shown in FIG. 16, the rotational screen unit 212 may be rotationally mounted on one side of the first gantry 150. If the subject 50 is scanned in the first gantry 150 in the feet-first direction, the rotational screen unit 212 may be open and the image projector 270 may project a content image onto the other face F of the rotational screen unit 212. On the other hand, if the subject 50 is scanned in the head-first direction, the rotational screen unit 212 is closed and the image projector 270 may stop projecting the content image.

In other words, the rotational screen unit 212 mounted on the first gantry 150 may be open to implement the out bore display when the subject 50 is scanned in the feet-first direction, and closed when the subject 50 is scanned in the head-first direction, thereby implementing no out bore display nor in bore display.

Opening or closing the rotational screen unit 212 may be performed in response to a user instruction input through the input unit 111. The image projector 270 may actively or passively project or stop projecting a content image in response to whether the rotational screen unit 212 is open or closed.

Active projecting or stopping projecting of a content image by the image projector 270 may be performed in response to whether the screen controller 222 detects the rotational screen unit 212 being open or closed. Specifically, when the rotational screen unit 212 is open, the screen controller 222 may detect this and generate a control signal to control the image projector 270 to project a content image. When the rotational screen unit 212 is closed, the screen controller 222 may detect this and generate a control signal to control the image projector 270 not to project or to stop projecting the content image.

Passive projection or stop projection of a content image by the image projector 270 may be performed by an instruction of the user through the input unit 111. Specifically, the user may input an instruction to start image projection through the input unit 111 when the user is aware that the rotational screen unit 212 is open, and then the image projector 270 may start projecting a content image in response to the user's instruction. Also, in response to the user's instruction to stop projection after the user is aware that the rotational screen unit 212 is closed, the image projector 270 may not project or may stop projecting the content image.

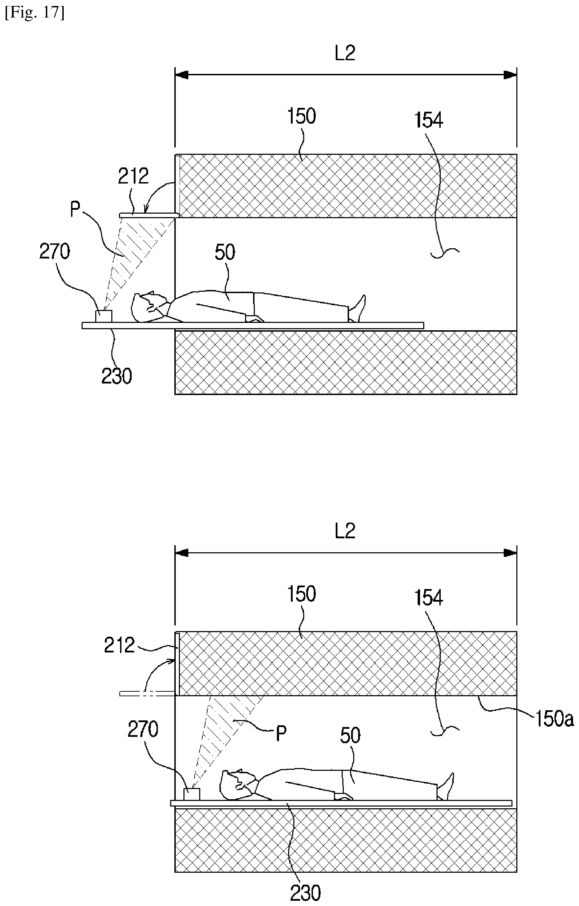

FIG. 17 shows an occasion where a rotational screen unit is mounted on the second gantry.

Referring to FIG. 17, the rotational screen unit 212 may be mounted on one side of the second gantry. As shown in the upper diagram of FIG. 17, in the case the head of the subject 50 is located outside the bore, the rotational screen unit 212 may be open and the image projector 270 may project a content image onto the rotational screen unit 212 that remains open. As shown in the lower diagram of FIG. 17, in the case the head of the subject 50 is located inside the bore, the rotational screen unit 212 may be closed. In this case, however, the image projector 270 may stop projecting the content image or keep on projecting the content image, depending on the system settings or the user's instruction through the input unit 111.

In other words, the rotational screen unit 212 mounted on the second gantry 150 may be open to implement the out bore display when the head of the subject 50 is located outside the bore, and closed when the head of the subject 50 is located inside the bore, thereby implementing no out bore display nor in bore display. In the latter case, however, the in bore display function may be performed when the inner wall 150a of the gantry reflects the projected image from the image projector 270. The second gantry 150 may implement both the out bore display and the in bore display.

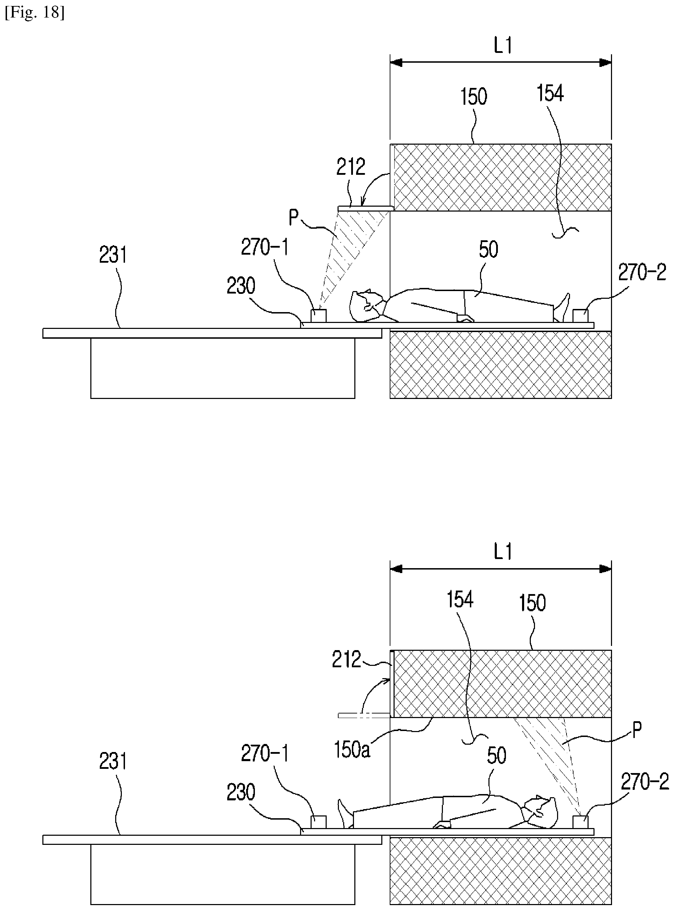

While the image projector 270 is implemented in singular form in the embodiment of FIG. 17, it may also be implemented in plural form in other embodiments. FIG. 18 shows a medical imaging apparatus having a first gantry on which a rotational screen unit is mounted, and a plurality of image projectors.

Referring to FIG. 18, the medical imaging apparatus 1 may include the first gantry on which the rotational screen unit 212 is mounted, and a plurality of image projectors 270-1, 270-2, e.g., a first image projector 270-1 and a second image projector 270-2 mounted on either end of the table 230.

As shown in the upper diagram of FIG. 18, in the case the subject 50 is scanned in the first gantry 150 in the feet-first direction, the rotational screen unit 212 may be open and the first image projector 270-1 may project a content image onto the rotational screen unit 212 that remains open. On the other hand, as shown in the lower diagram of FIG. 18, in the case the subject 50 is scanned in the head-first direction, the rotational screen unit 212 may be closed and the first image projector 270-1 may stop projecting the content image. In this case, the second image projector 270-2 may project a content image, which is reflected off an inner wall 150a of the gantry 150.

In other words, the rotational screen unit 212 mounted on the first gantry 150 may be open to implement the out bore display when the subject 50 is scanned in the feet-first direction, and closed when the subject 50 is scanned in the head-first direction, thereby implementing no out bore display nor in bore display. In the latter case, however, the in bore display function may be performed when the inner wall 150a of the gantry reflects the projected image from the second image projector 270-2. In other words, with the multiple image projectors 270, both the out bore display and the in bore display may be implemented.

Similar to the image projector 270 implemented in plural form as shown in FIG. 18, the screen unit 210 may also be implemented in plural form. For example, the screen unit 210 may be implemented with a plurality of rotational screen units 212.

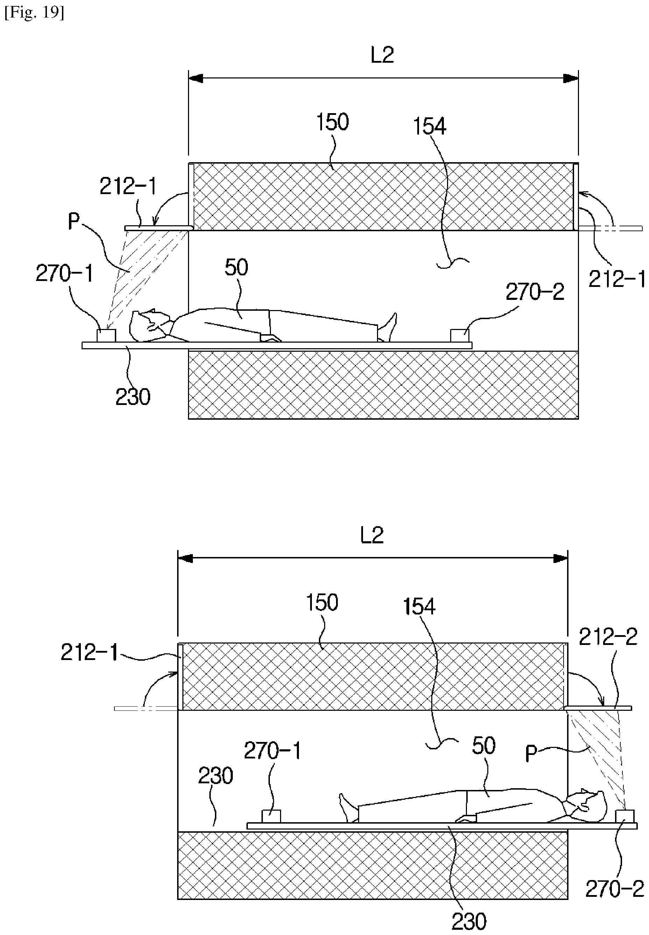

FIG. 19 shows a medical imaging apparatus having the second gantry on which a plurality of rotational screen units are mounted, and a plurality of image projectors.

Referring to FIG. 19, the medical imaging apparatus 1 may include the second gantry on which rotational screen units are mounted, and first and second image projectors 270-1 and 270-2 mounted on either end of the table 230. The rotational screen units 212-1, 212-2 may be mounted outside the gantry 150 on either side, the one mounted on the left side called a first rotational screen unit 212-1 and the other one mounted on the right side called a second rotational screen unit 212-2.

As shown in the upper diagram of FIG. 19, in the case the head of the subject 50 is located outside the bore on the left, the first rotational screen unit 212-1 may be open and the first image projector 270-1 may project a content image onto the first rotational screen unit 212-1 that remains open. In this case, the second rotational screen unit 212-2 located on the right may get closed or remain closed.

As shown in the lower diagram of FIG. 19, in th case the head of the subject 50 is located outside the bore on the right, the second rotational screen unit 212-2 may be open and the second image projector 270-2 may project a content image onto the second rotational screen unit 212-2 that remains open. In this case, the first rotational screen unit 212-1 located on the left may get closed or remain closed.

In other words, the first rotational screen unit 212-1 mounted on the second gantry 150 may be open to implement the out bore display when the head of the subject 50 is located outside the bore on the left, and the second rotational screen unit 212-2 may be open to implement the out bore display when the head of the subject 50 is located outside of the bore on the right. If the head of the subject 50 is located inside the bore, the plurality of rotational screen units 212-1, 212-2 may all be closed. However, the in bore display function may be performed when the inner wall 150a of the gantry reflects the projected image from the first or second image projector 270-1 or 270-2. The second gantry 150 may implement both the out bore display and the in bore display.

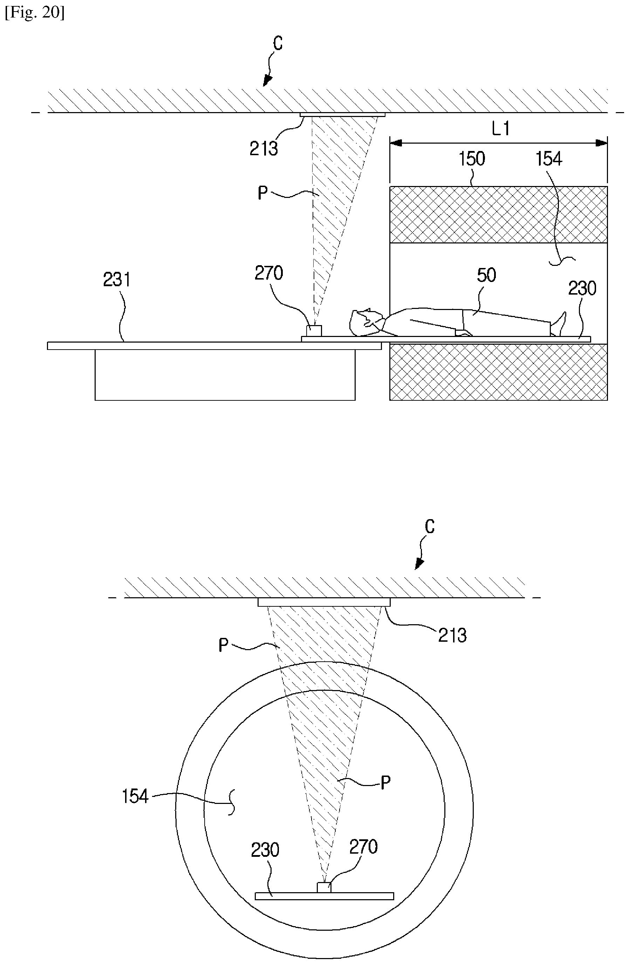

FIG. 20 is a diagram for explaining a screen unit, according to another embodiment of the present disclosure.

Referring to FIG. 20, a screen unit 213 may be arranged in the way that is fixed on the ceiling C of the examination room, which will now be referred to as a fixed screen unit.

The fixed screen unit 213 may be formed of a film or panel and mounted or glued to the ceiling C. Alternatively, the ceiling C itself may be formed of a material that may reflect a projected image, thus forming the fixed screen unit 213. The fixed screen unit 213 may be formed such that it may cover a field of view of the subject 50 when the head of the subject 50 is located outside the gantry 150.

As shown in FIG. 20, the fixed screen unit 213 may be situated outside and above the first gantry 150. If the subject 50 is scanned in the first gantry 150 in the feet-first direction, the image projector 270 may project a content image and the fixed screen unit 213 may display the projected image to be watched by the subject 50. On the other hand, if the subject 50 is scanned in the head-first direction, the image projector 270 may stop projecting the content image and accordingly, the fixed screen unit 213 may not display the content image.

In other words, the fixed screen unit 213 arranged above the first gantry 150 may implement the out bore display when the subject 50 is scanned in the feet-first direction, and may implement no out bore display nor in bore display when the subject 50 is scanned in the head-first direction.

The image projector 270 may project or stop projecting the content image in response to the user's instruction through the input unit 111. Specifically, the user may input an instruction to start image projection through the input unit 111 when the user is aware that the subject 50 is scanned in the the feet-first direction, and then the image projector 270 may start projecting a content image in response to the user's instruction. Also, in response to the user's instruction to stop projection after the user is aware that the subject 50 is scanned in the head-first direction, the image projector 270 may not project or may stop projecting the content image.

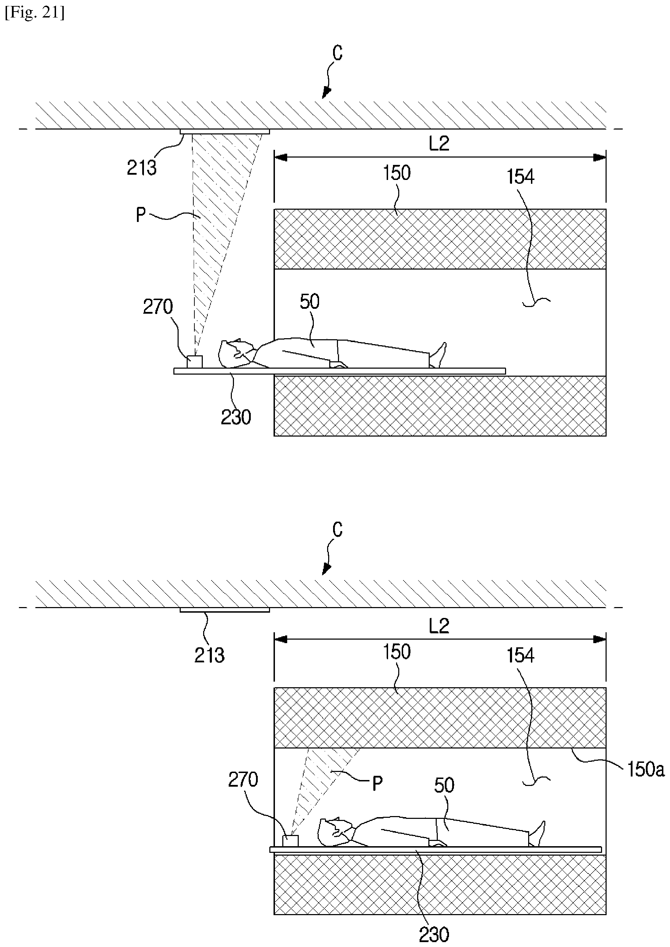

FIG. 21 shows an occasion where a fixed screen unit is arranged above the second gantry.

Referring to FIG. 21, the fixed screen unit 213 may be arranged outside and above the second gantry. As shown in the upper diagram of FIG. 21, in the case the head of the subject 50 is located outside the bore, the image projector 270 may project a content image and the fixed screen unit 213 located to face the image projector 270 may display the projected image.

As shown in the lower diagram of FIG. 21, if the head of the subject 50 is located inside the bore, the image projector 270 may stop projecting the content image or keep on projecting the content image, depending on the system settings or the user's instruction through the input unit 111. However, even if the image projector 270 is projecting the content image, the fixed screen unit 213 not located to face the image projector 270 is unable to display the content image. In this case, the projected image may be displayed by being reflected off the inner wall 150a of the gantry.

In other words, the fixed screen unit 213 arranged above the second gantry 150 may implement the out bore display when the head of the subject 50 is located outside the bore, and implement no out bore display nor in bore display when the head of the subject 50 is located inside the bore. In the latter case, however, the in bore display function may be performed when the inner wall 150a of the gantry reflects the projected image from the image projector 270. The second gantry 150 may implement both the out bore display and the in bore display.

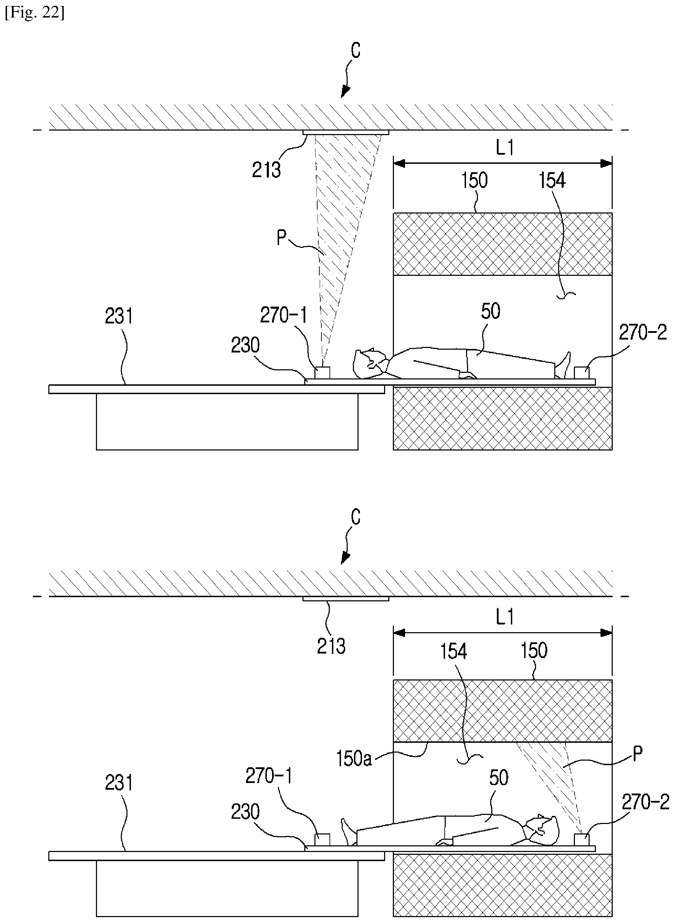

While the image projector 270 is implemented in singular form in the embodiment of FIG. 21, it may also be implemented in plural form in other embodiments. FIG. 22 shows a medical imaging apparatus having a fixed screen unit and a plurality of image projectors.

Referring to FIG. 22, the medical imaging apparatus 1 may include the fixed screen unit 213 arranged outside and above the first gantry 150, and a plurality of image projectors 270-1, 270-2, e.g., the first image projector 270-1 and the second image projector 270-2 mounted on either end of the table 230.

As shown in the upper diagram of FIG. 22, if the subject 50 is scanned in the first gantry 150 in the feet-first direction, the first image projector 270-1 may project a content image, which may in turn be reflected off and displayed on the fixed screen unit 213 located to face the first image projector 270-1. On the other hand, as shown in the lower diagram of FIG. 22, if the subject 50 is scanned in the head-first direction, the first image projector 270-1 may stop projecting the content image. In this case, the second image projector 270-2 may project a content image, which is reflected off the inner wall 150a of the gantry 150.

In other words, the fixed screen unit 213 arranged above the the first gantry 150 may implement the out bore display when the subject 50 is scanned in the feet-first direction, and implementing no out bore display nor in bore display when the subject 50 is scanned in the head-first direction. In the latter case, however, the in bore display function may be performed when the inner wall 150a of the gantry reflects the projected image from the second image projector 270-2. In this regard, the multiple image projectors 270 may enable both the out bore display and the in bore display.

As shown in FIG. 22, the image projector 270 may be implemented in plural form, and likewise, the screen unit 210 may be implemented in plural form. The screen unit 210, for example, may be implemented with a plurality of fixed screens 213.

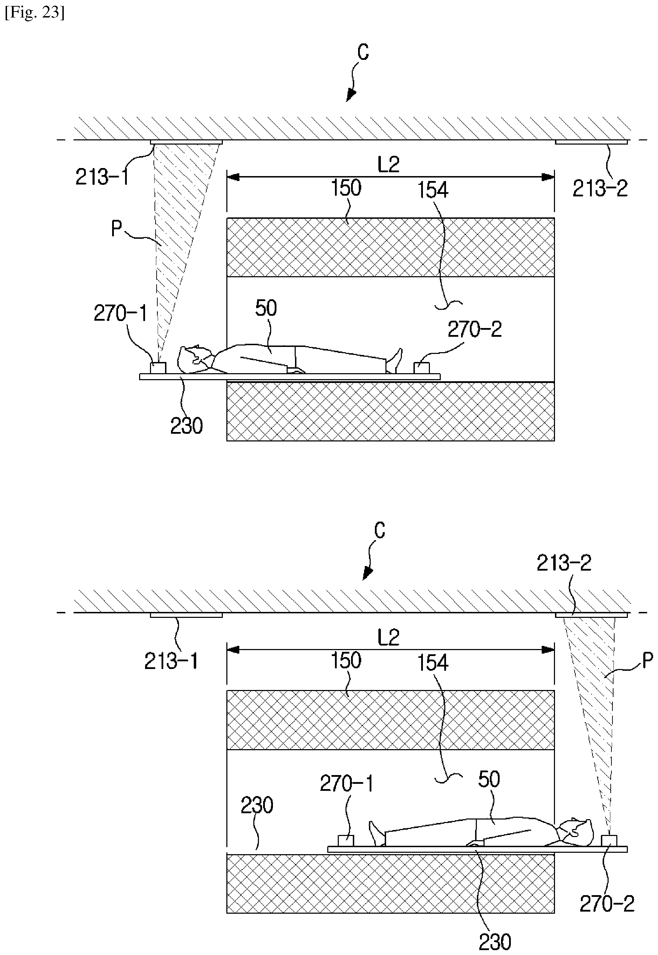

FIG. 23 shows a medical imaging apparatus having a plurality of fixed screen units and a plurality of image projectors.

Referring to FIG. 23, the medical imaging apparatus 1 may include a plurality of fixed screen units 213-1, 213-2, and first and second image projectors 270-1 and 270-2 mounted on either end of the table 230. The fixed screen units 213-1, 213-2 may be arranged above the gantry 150 on either side, the one arranged on the left side called a first fixed screen unit 213-1 and the other one arranged on the right side called a second fixed screen unit 213-2.

As shown in the upper diagram of FIG. 23, in the case the head of the subject 50 is located outside the bore on the left, the first image projector 270-1 may project a content image, which may be reflected off and displayed on the first fixed screen unit 213-1 located to face the first image projector 270-1. In this case, the second image projector 270-2 may not start projecting or may stop projecting the content image, and the second fixed screen unit 213-2 may not display the content image.

As shown in the upper diagram of FIG. 23, in the case the head of the subject 50 is located outside the bore on the right, the second image projector 270-2 may project a content image, which may be reflected off and displayed on the second fixed screen unit 213-2 located to face the second image projector 270-2. In this case, the first image projector 270-1 may not start projecting or may stop projecting the content image, and the first fixed screen unit 213-1 may not display the content image.

In other words, the first fixed screen unit 213-1 arranged on the left of the second gantry 150 may implement the out bore display when the head of the subject 50 is located outside the bore on the left, and the second fixed screen unit 213-2 arranged on the right of the second gantry 150 may implement the out bore display when the head of the subject 50 is located outside of the bore on the right. In the case the head of the subject 50 is located inside the bore, the plurality of fixed screen units 213-1, 213-2 may not implement the out bore display nor the in bore display. However, the in bore display function may be performed when the inner wall 150a of the gantry reflects the projected image from the first or second image projector 270-1 or 270-2. The second gantry 150 may implement both the out bore display and the in bore display.

Passive implementation of the out bore display by the screen unit 210 has thus far been described. To sum up, pushing or pulling the push/pull screen unit 211, opening or closing the rotational screen unit 212, or projecting or stop projecting a content image onto the fixed screen unit 213 may be performed in response to an instruction of the user through the input unit 111. On the other end, with various sensors for the medical imaging apparatus 1, the screen unit 210 may actively implement the out bore display.

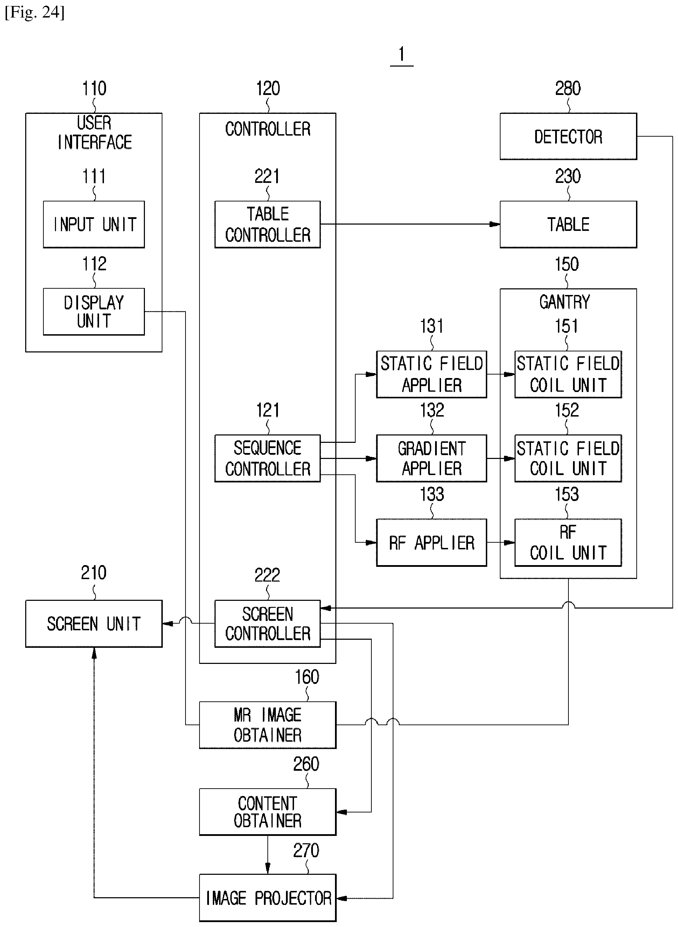

As discussed above, the medical imaging apparatus 1 may include the image projector 270 and the screen unit 210, for providing images to the subject in various ways. The image projector 270 and the screen unit 210 onto which an image is projected may be controlled by the user, or according to a predetermined protocol, or based on where the subject is. Controlling the image projector 270 and the screen unit 210 will now be described in detail in connection with FIGS. 24 to 38.

FIG. 24 is a control block diagram of a medical imaging apparatus, according to another embodiment of the present disclosure.

Referring to FIG. 24, the medical imaging apparatus 1 may include the user interface 110 for providing a user interface, the table 230 for carrying a subject, the gantry 150 for producing a magnetic field and receiving a magnetic resonance signal produced from the subject, the MR image obtainer 160 for performing various processes on the magnetic resonance signal to obtain a magnetic resonance image, the content obtainer 260 for obtaining various content to be provided for the subject, the image projector 270 for projecting images contained in the content, the screen unit 210 to display the projected image, a detector 280 for detecting the location of the table or the location of the subject, and the controller 120 for controlling overall operation of the medical imaging apparatus 1. The same components of the medical imaging apparatus 1 as described above will be omitted herein.

The detector 280 may detect the location of the subject 50. The screen controller 222 may provide an image for the subject by sending control signals to the screen unit 210 and the image projector 270 based on the location of the subject 50 detected by the detector 280. The location of the subject 50 may include a location where the subject 50 lies, a location of the head of the subject 50, and a location of a gaze of the subject 50.

To detect the location, the detector 280 may include at least one of table location sensor, subject location sensor, and eyeball tracking sensor.

The table location sensor refers to a sensor for detecting a table movement location, and the screen controller 222 may detect the location of the subject based on the table movement location detected by the table location sensor. The table location sensor may be implemented with, e.g., an image sensor, a switch, a photo sensor, etc.

The subject location sensor refers to a sensor for detecting an exact position of the subject 50, such as a position or direction in which the subject 50 lies on the table 230, and may be implemented with an image sensor, a weight sensor, etc.

The eyeball tracking sensor refers to a sensor for detecting a gaze of the subject 50 from eyeball positions, and may be implemented with e.g., an image sensor. The form in which the sensors are implemented are not limited thereto, but may have any different forms well-known in the art.

The controller 120 may include the table controller 221 for controlling the movement of the table 230, the sequence controller 121 for planning a pulse sequence or controlling operation of the gantry 150 according to the pulse sequence, the content obtainer 160 for obtaining and providing content for the subject, and the screen controller 222 for controlling the image projector 270 and the screen unit 210. The screen controller 222 may receive a sensed value from the detector 280, and based on the sensed value, control the image projector 270 or screen unit 210.

Specifically, the screen controller 222 may control the image projector 270 to provide an image for the subject, based on the position of the subject detected by the detector 280. For example, the screen controller 222 may control the image projector 270 to change the location for an image to be projected based on the position of the subject, or control the image projector 270 to be focused based on the location of the screen unit 210 for the image to be projected.

In addition, the screen controller 222 may control the screen unit 210 for the image to be projected to be moved (e.g., pulled out, pushed in, or rotated) based on the position of the subject, or determine the location of the screen unit 210 for the image to be projected based on the position of the subject.

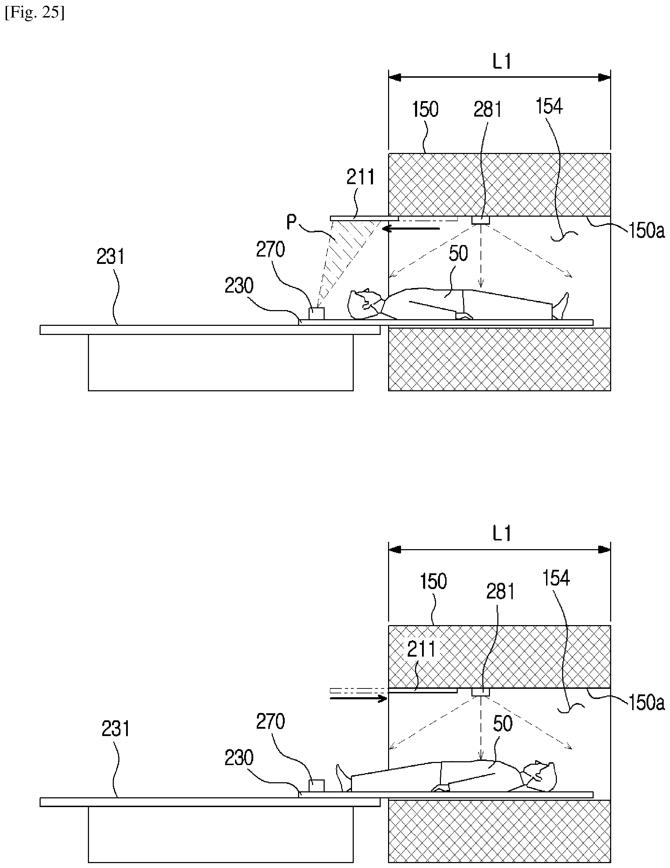

FIG. 25 shows a first gantry on which a push/pull screen unit and an image sensor are mounted.

Referring to FIG. 25, the medical imaging apparatus 1 may include the first gantry 150 on which the push/pull screen unit 211 is mounted on one side, and the first gantry 150 may include an image sensor 281, such as a camera. The image sensor 281 may be arranged on the inner wall 150a of the first gantry 150 for capturing the subject. The screen controller 222 may receive a captured image from the image sensor 281, and detect a position of the subject, e.g., a location where the subject 50 lies, or a location of the head of the subject 50 from the captured image.

As shown in the upper diagram of FIG. 25, if the subject is lying in the feet-first direction, the screen controller 222 may detect based on the captured image from the image sensor 281 that the head of the subject 50 is outside the bore, and based on this, generate a control signal for pulling out the push/pull screen unit 211 and a control signal for image projection of the image projector 270. The push/pull screen unit 211 may be pulled out of the gantry 150 according to the control signal of the screen controller 222, implementing the out bore display. At this time, the screen controller 222 may output a control signal to the image projector 270 for an image to be projected, in response to the push/pull screen unit 211 being pulled out. The image projector 270 may control a location for the image to be projected based on the control signal, or control the focus of the projected image for clear projection.

On the other hand, as shown in the lower diagram of FIG. 25, if the subject is lying in the head-first direction, the screen controller 222 may detect based on the captured image from the image sensor 281 that the head of the subject 50 is inside the bore, and based on this, generate a control signal for pushing in the push/pull screen unit 211 and a control signal to stop image projection of the image projector 270. The push/pull screen unit 211 may be pushed into the gantry 150 according to the control signal of the screen controller 150, thus not implementing the out bore display (nor the in bore display).

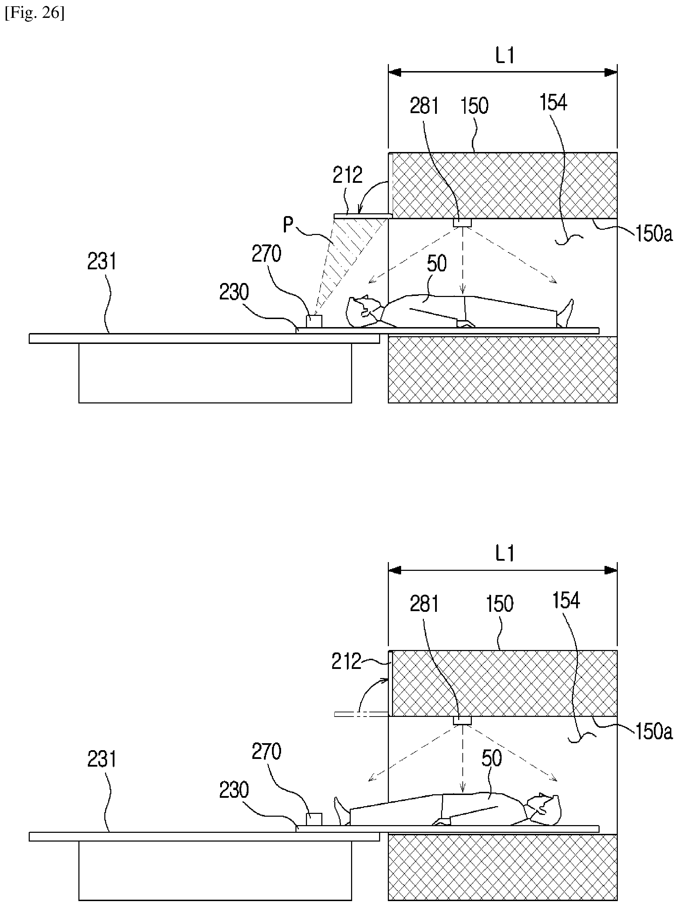

FIG. 26 shows a first gantry on which a rotational screen unit and an image sensor are mounted.

Referring to FIG. 26, the medical imaging apparatus 1 may include the first gantry 150 on which the rotational screen unit 212 is mounted on one side, and the first gantry 150 may include an image sensor 281 mounted on the upper inner wall 150a for capturing the subject.

As shown in the upper diagram of FIG. 26, if the subject is lying in the feet-first direction, the screen controller 222 may detect based on the captured image from the image sensor 281 that the head of the subject 50 is outside the bore, and based on this, generate a control signal for opening the rotational screen unit 212 and a control signal for image projection of the image projector 270. The rotational screen unit 212 may be open according to the control signal of the screen controller 222, implementing the out bore display. The image projector 270 may control the location for the image to be projected such that the image is projected onto the rotational screen unit 212 which is open, based on the control signal, and control the focus of the projected image for clear projection.

On the other hand, as shown in the lower diagram of FIG. 26, if the subject is lying in the head-first direction, the screen controller 222 may detect based on the captured image from the image sensor 281 that the head of the subject 50 is inside the bore, and based on this, generate a control signal for closing the rotational screen unit 212 and a control signal to stop image projection of the image projector 270. The rotational screen unit 212 may be closed according to the control signal of the screen controller 150, thus not implementing the out bore display (nor the in bore display).

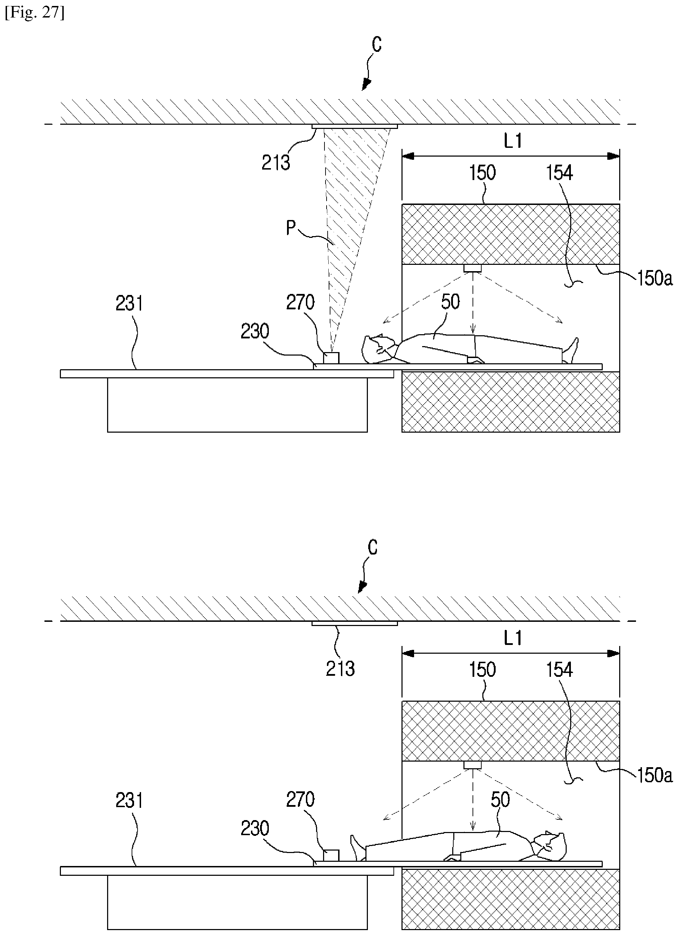

FIG. 27 shows a first gantry with a fixed screen unit arranged above the first gantry and an image sensor.

Referring to FIG. 27, the medical imaging apparatus 1 may include the first gantry 150, and there may be the fixed screen unit 213 arranged outside and above the first gantry 150. The image sensor 281 may be mounted on the inner wall 150a of the first gantry 150 for capturing the subject.

As shown in the upper diagram of FIG. 27, if the subject is lying in the feet-first direction, the screen controller 222 may detect based on the captured image from the image sensor 281 that the head of the subject 50 is outside the bore, and based on this, generate a control signal for image projection of the image projector 270. The image projector 270 may project a content image according to the control signal of the screen controller 150, and the fixed screen unit 213 may implement the out bore display.

On the other hand, as shown in the lower diagram of FIG. 27, if the subject is lying in the head-first direction, the screen controller 222 may detect based on the captured image from the image sensor 281 that the head of the subject 50 is outside the bore, and based on this, generate a control signal to stop image projection of the image projector 270. The image projector 270 may not project the content image or may stop projecting the content image according to the control signal from the screen controller 150, and the fixed screen unit 213 may not implement the out bore display (nor the in bore display).

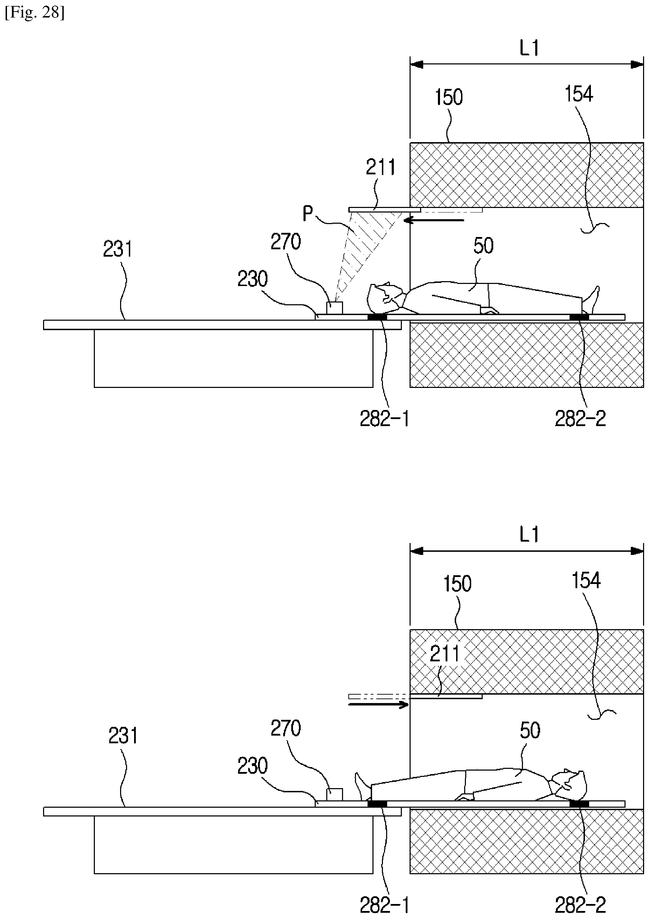

FIG. 28 shows an example of a medical imaging apparatus including a first gantry and a weight sensor.

Referring to FIG. 28, the medical imaging apparatus 1 may include the first gantry 150 on which the push/pull screen unit 211 is mounted on one side. The medical imaging apparatus 1 may also include the table 230 on which at least one of weight sensors 282-1, 282-2 are mounted.

The weight sensors 282-1, 282-2 may include a first weight sensor 282-1 and a second weight sensor 282-2, one arranged on the same side where the image projector 270 is located and the other one on the opposite side with respect to the center of the table 230. For example, the first weight sensor 282-1 may be arranged on the side where the image projector 270 is located, and the second weight sensor 282-2 may be arranged on the opposite side.

Depending on the direction in which the subject 50 is lying down, the first weight sensor 281-1 may measure a heavier weight than a weight measured by the second weight sensor 282-2, or the other way around. The screen controller 222 may receive the measurements from the first and second weight sensors 282-1, 282-2, and compare the two measurements to detect a location where the subject 50 is lying, a location of the head of the subject 50, or the like.

The screen controller 222 may control the screen unit 210 and the image projector 270 to provide an image for the subject based on the position of the subject detected based on the detection result of the weight sensors 282-1, 282-2.

As shown in the upper diagram of FIG. 28, if the subject is lying in the feet-first direction, the first weight sensor 282-1 may measure a heavier weight than the second weight sensor 282-2 may do. The screen controller 222 may detect that the head of the subject 50 is outside the bore based on the measurements of the weight sensors 282-1, 282-2, and based on this, generate a control signal to pull out the push/pull screen unit 211 and a control signal for image projection of the image projector 270. The push/pull screen unit 211 may be pulled out of the gantry 150 according to the control signal of the screen controller 150, implementing the out bore display.

As shown in the lower diagram of FIG. 28, if the subject is lying in the head-first direction, the second weight sensor 282-2 may measure a heavier weight than the first weight sensor 282-1 may do. The screen controller 222 may detect that the head of the subject 50 is inside the bore based on the measurements of the weight sensors 282-1, 282-2, and based on this, generate a control signal to push in the push/pull screen unit 211 and a control signal to stop projection of the image projector 270. The push/pull screen unit 211 may be pushed into the gantry 150 according to the control signal of the screen controller 150, thus not implementing the out bore display (nor the in bore display).

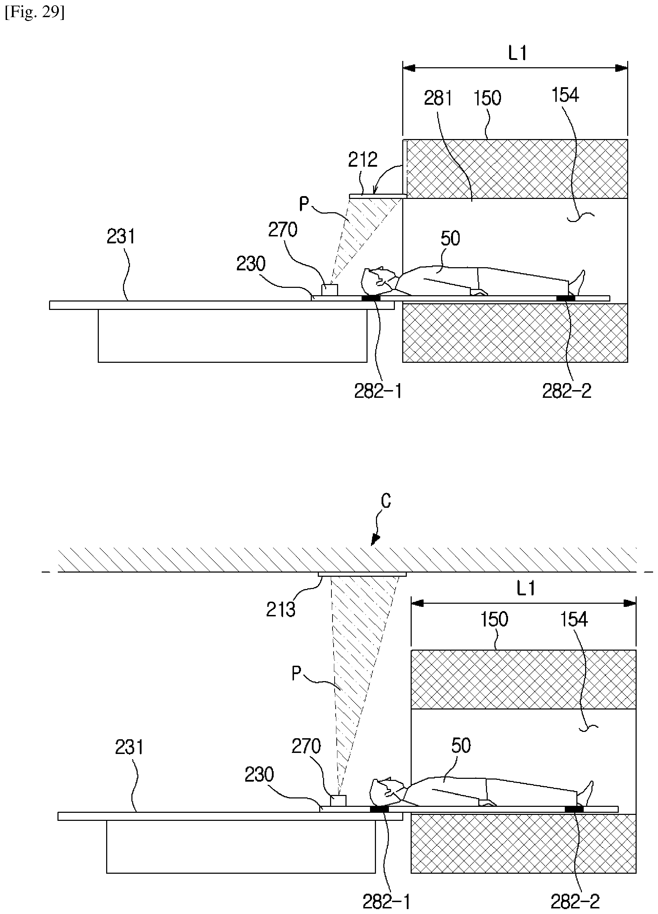

FIG. 29 shows another example of a medical imaging apparatus including a first gantry and a weight sensor.

Referring to FIG. 29, the medical imaging apparatus 1 may include a first gantry, and the table 230 on which the first and second weight sensors 282-1, 282-2 are mounted. The first weight sensor 282-1 may be mounted on the same side where the image projector 270 is located with respect to the center of the table 230, and the second weight sensor 282-2 may be mounted on the opposite side. That is, the first weight sensor 282-1 may measure a weight on the side where the image projector 270 is located, and the second weight sensor 282-2 may measure a weight on the opposite side.

As shown in FIG. 29, if the subject is lying in the feet-first direction, the first weight sensor 282-1 may measure a heavier weight than the second weight sensor 282-2 may do. The screen controller 222 may detect from the measurements of the weight sensors 282-1, 282-2 that the head of the subject 50 is outside the bore.

As shown in the upper diagram of FIG. 29, in a case the rotational screen unit 212 is mounted on one side of the first gantry 150, the screen controller 222 may generate a control signal to open the rotational screen unit 212 and a control signal for image projection of the image projector 270, based on the detected head location. The rotational screen unit 212 may be open according to the control signal of the screen controller 150, implementing the out bore display.

As shown in the lower diagram of FIG. 29, in a case the fixed screen unit 212 is arranged outside and above the first gantry 150, the screen controller 222 may control the image projector 270 to project a content image based on the detected head location. Accordingly, the fixed screen unit 213 may display the projected image, thus implementing the out bore display.

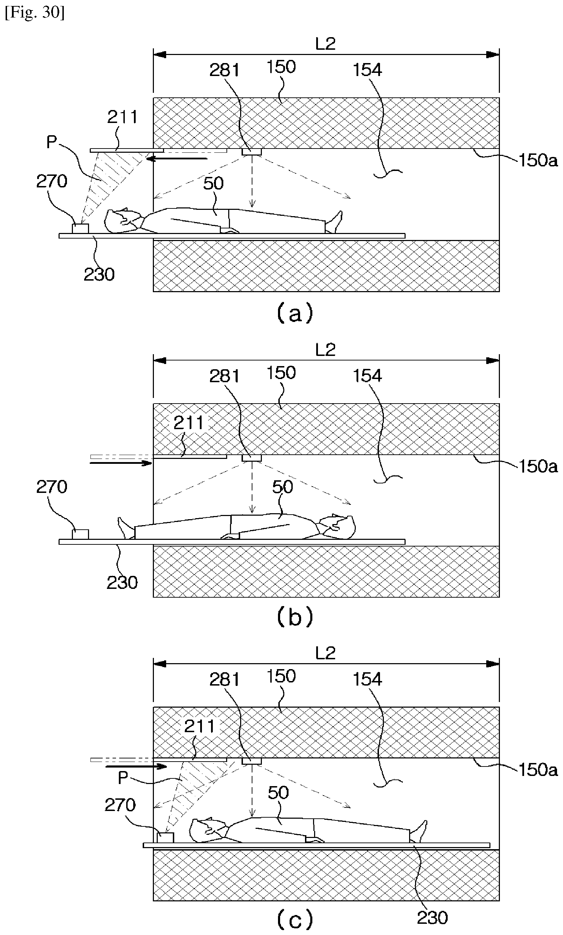

FIG. 30 shows a second gantry on which a push/pull screen unit and an image sensor are mounted.

Referring to FIG. 30, the medical imaging apparatus 1 may include the second gantry 150 on which the push/pull screen unit 211 is mounted on one side, and the second gantry 150 may include an image sensor 281, such as a camera. The image sensor 281 may be arranged on the inner wall 150a of the second gantry 150 for capturing the table 230 and the subject 50. The screen controller 222 may receive a captured image from the image sensor 281, and detect a location to which the table 230 has been moved, and a position of the subject, e.g., a location where the subject 230 lies, or a location of the head of the subject 50, from the captured image.

As shown in (a) of FIG. 30, the screen controller 222 may detect that a part of the table 230, i.e., the head of the subject 50 is located outside the bore based on the captured image from the image sensor 281, and based on this, generate a control signal to pull out the push/pull screen unit 211 and a control signal for image projection of the image projector 270. The push/pull screen unit 211 may be pulled out of the gantry 150 according to the control signal of the screen controller 150, implementing the out bore display. At this time, the image projector 270 may adjust the focus or location for an image to be projected according to the control signal.

As shown in (b) of FIG. 30, the screen controller 222 may detect that a part of the table 230, i.e., the head of the subject 50 is located inside the bore based on the captured image from the image sensor 281, and based on this, generate a control signal to push in the push/pull screen unit 211 and a control signal to stop projection for the image projector 270. The push/pull screen unit 211 may be pushed into the gantry 150 according to the control signal of the screen controller 150, thus not implementing the out bore display (nor the in bore display).

As shown in (c) of FIG. 30, the screen controller 222 may detect based on the captured image from the image sensor 281 that the table 230 and the subject 50 are all inside the bore and the head of the subject 50 is located on the same side as the image projector 270. Based on this, the screen controller 222 may generate a control signal to push in the push/pull screen unit 211 and a control signal for image projection of the image projector 270. According to the control signal of the screen controller 222, the push/pull screen unit 211 may be pushed in, and thus may not implement the out bore display, but may implement the in bore display.

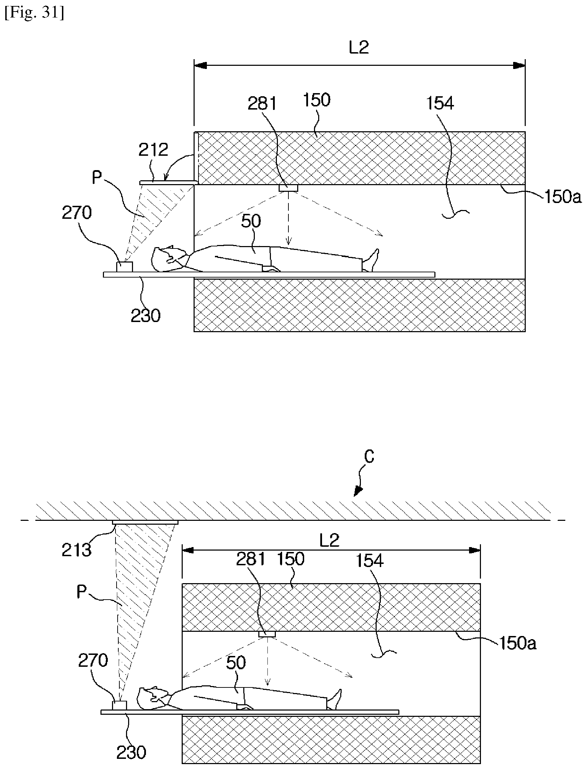

FIG. 31 shows a second gantry on which an image sensor is mounted.

Referring to FIG. 31, the medical imaging apparatus 1 may include the second gantry 150 having an image sensor arranged on the upper inner wall 150a for capturing the table 230 and the subject 50. The screen controller 222 may detect based on the captured image from the image sensor 281 that a part of the table 230, i.e., a part where the head of the subject 50 is located, is outside the bore.

As shown in the upper diagram of FIG. 31, in the case the rotational screen unit 212 is mounted on one side of the second gantry 150, the screen controller 222 may generate a control signal to open the rotational screen unit 212 and a control signal for image projection of the image projector 270, based on the detection result. The rotational screen unit 212 may be open according to the control signal of the screen controller 150, implementing the out bore display. At this time, the image projector 270 may adjust the focus or location for an image to be projected according to the control signal.

As shown in the lower diagram of FIG. 31, in the case the fixed screen unit 212 is arranged outside and above the second gantry 150, the screen controller 222 may control the image projector 270 to project a content image based on the detection result. Accordingly, the fixed screen unit 213 may display the projected image, thus implementing the out bore display.

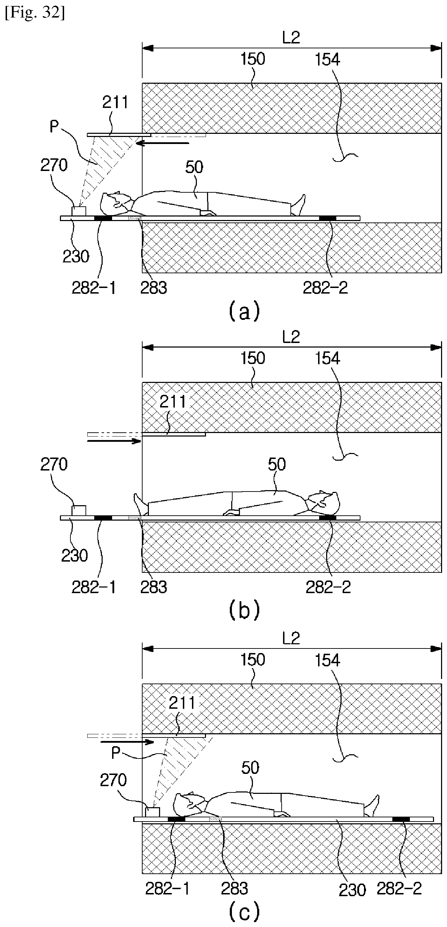

FIG. 32 shows a medical imaging apparatus including a second gantry, a switch, and a weight sensor.

Referring to FIG. 32, the medical imaging apparatus 1 may include the second gantry 150 on which the push/pull screen unit 211 is mounted on one side. The medical imaging apparatus 1 may also include the table 230 on which a switch 283 and at least one of weight sensors 282-1, 282-2 are mounted. The switch 283 may be a contact switch, such as a limit switch, a micro switch, a touch switch, etc., or a contactless switch, such as a photoelectric switch, an ultrasound switch, etc.

The switch 283 may be mounted on the same side where the image projector 270 is located, with respect to the center of the table 230. Accordingly, the switch 283 may be turned on or off, depending on whether a part of the table 230 where the image projector 270 is located is outside or inside the bore. For example, the switch 283 may be turned on when the part of the table 230 where the image projector 270 is located is outside the bore, and turned off when the part is inside the bore.

The weight sensors 282-1, 282-2 may include a first weight sensor 282-1 and a second weight sensor 282-2, the first weight sensor 282-1 arranged on the same side where the image projector 270 is located with respect to the center of the table 230, and the second weight sensor 282-2 arranged on the opposite side. That is, the first weight sensor 282-1 may measure a weight on the side where the image projector 270 is located, and the second weight sensor 282-2 may measure a weight on the opposite side. Depending on the direction in which the subject 50 is lying down and the location of the head of the subject 50, the first weight sensor 281-1 may measure a heavier weight than the second weight sensor 282-2 may do, or the other way around.

The screen controller 222 may detect a location to which the table 230 has been moved, from the state of the switch 283, and compare the measurements of the first and second weight sensors 282-1 and 282-2 to detect a position of the subject 50, such as a location where the subject 50 is lying or a location of the head of the subject 50.

The screen controller 222 may control the screen unit 210 for an image to be projected to be moved (e.g., pulled out, pushed in, or rotated) based on the detected position of the subject with the switch 283, the first and second weight sensors 282-1, 282-2, or determine a location of the screen unit 210 for the image to be projected based on the position of the subject. The screen controller 222 may also control the image projector 270 based on a change in location of the screen unit 210 corresponding to the location of the subject. For example, the screen controller 222 may control a location for an image to be projected or a focus of the projected image for clear projection based on the change in location of the screen unit 210.

As shown in (a) of FIG. 32, the switch 283 may be turned on, and the first weight sensor 282-1 may measure a relatively heavy weight as compared with the second weight sensor 282-2. The screen controller 222 may detect from the turned-on state of the switch 283 that a part of the table 230 where the image projector 270 is located is outside the bore, and detect from the measurements of the weight sensors 282-1, 282-2 that the head of the subject 50 is located on the same side where the image projector 270 is located. Then, the screen controller 222 may detect that the head of the subject 50 is outside the bore, and based on this, generate a control signal to pull out the push/pull screen unit 211 and a control signal for image projection of the image projector 270. The push/pull screen unit 211 may be pulled out of the gantry 150 according to the control signal of the screen controller 150, implementing the out bore display.

As shown in (b) of FIG. 32, the switch 283 may be turned on, and the second weight sensor 282-2 may measure a relatively heavy weight as compared with the first weight sensor 282-1. Based on this, the screen controller 222 may detect that the head of the subject 50 is inside the bore although a part of the table 230 is outside the bore, and generate a control signal to push in the push/pull screen unit 211 and a control signal to stop projection of the image projector 270. The push/pull screen unit 211 may be pushed into the gantry 150 according to the control signal of the screen controller 150, thus not implementing the out bore display (nor the in bore display).

As shown in (c) of FIG. 32, the switch 283 may be turned on, and the first weight sensor 282-1 may measure a relatively heavy weight as compared with the second weight sensor 282-2. Based on this, the screen controller 222 may detect that the table 230 and the subject 50 are all inside the bore and the head of the subject 50 is located on the same side where the image projector 270 is located. The screen controller 222 may generate a control signal to push in the push/pull screen unit 211 and a control signal for image projection of the image projector 270. According to the control signal of the screen controller 222, the push/pull screen unit 211 may be pushed in, and thus may not implement the out bore display but may implement the in bore display.

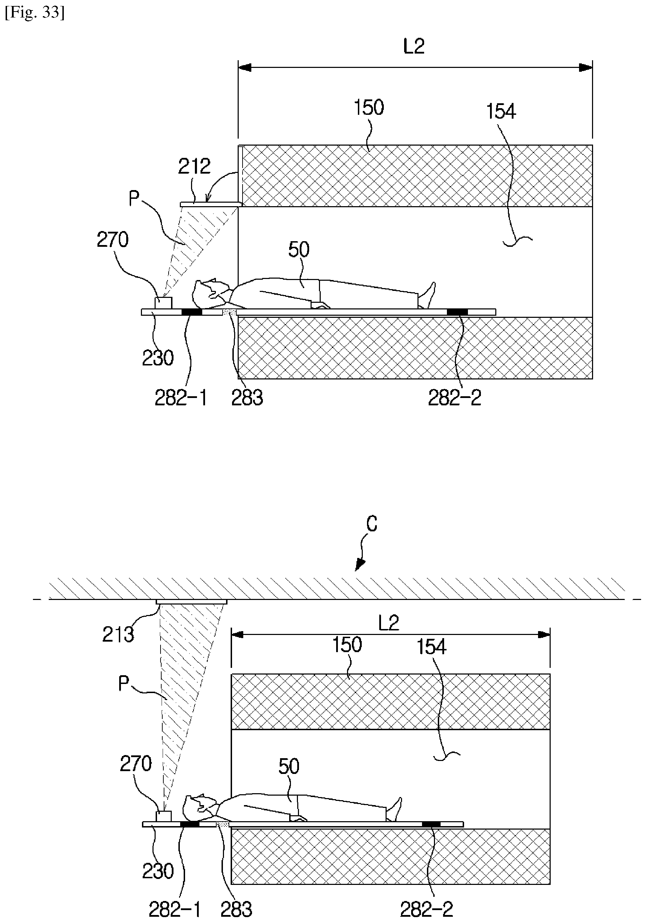

FIG. 33 shows a medical imaging apparatus including a switch and weight sensors, according to an embodiment of the present disclosure.

Referring to FIG. 33, the medical imaging apparatus 1 may include a second gantry 150, and the table 230 on which the switch 283, and the first and second weight sensors 282-1, 282-2 are mounted. Positions or operations of the switch 283 and the first and second weight sensors 282-1, 282-2 are assumed to be the same as described above in connection with FIG. 32.

Accordingly, the switch 283 may be turned on, and the first weight sensor 282-1 may measure a relatively heavy weight as compared with the second weight sensor 282-2. The screen controller 222 may detect from the turned-on state of the switch 283 that a part of the table 230 where the image projector 270 is located is outside the bore, and detect from the measurements of the weight sensors 282-1, 282-2 that the head of the subject 50 is located on the same side where the image projector 270 is located. That is, the screen controller 222 may detect that the head of the subject 50 is outside the bore.

As shown in the upper diagram of FIG. 33, in the case the rotational screen unit 212 is mounted on one side of the second gantry 150, the screen controller 222 may generate a control signal to open the rotational screen unit 212 and a control signal for image projection of the image projector 270, based on the detection result. The rotational screen unit 212 may be open according to the control signal of the screen controller 150, implementing the out bore display.

As shown in the lower diagram of FIG. 33, in the case the fixed screen unit 212 is arranged outside and above the second gantry 150, the screen controller 222 may control the image projector 270 to project a content image based on the detection result. Accordingly, the fixed screen unit 213 may display the projected image, thus implementing the out bore display.

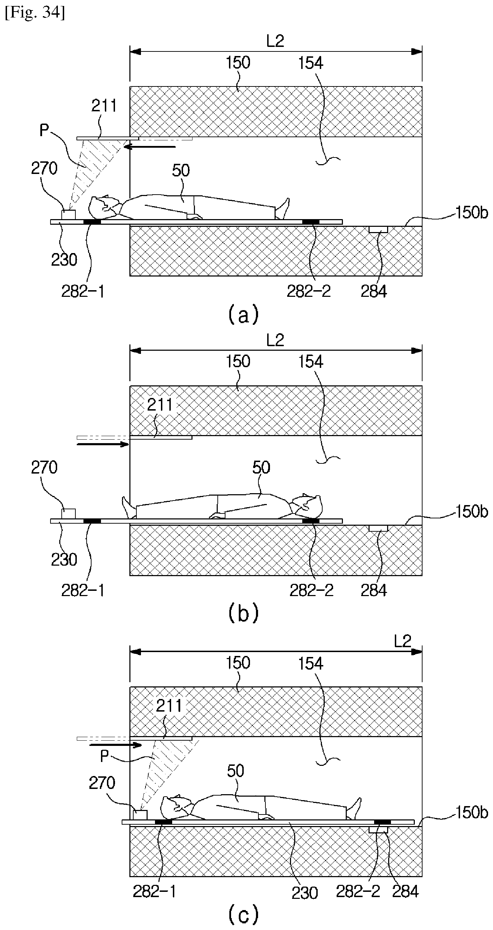

FIG. 34 shows a medical imaging apparatus including a second gantry, a photo sensor, and weight sensors, according to an embodiment of the present disclosure.

Referring to FIG. 34, the medical imaging apparatus 1 may include the second gantry 150 on which the push/pull screen unit 211 is mounted on one side, and the second gantry 150 may include a photo sensor 284. The medical imaging apparatus 1 may also include the table 230 on which the photo sensor 283 and at least one of weight sensors 282-1, 282-2 are mounted.

The photo sensor 284 may be mounted on the lower inner wall 150b of the second gantry 150, for irradiating light upward, receiving reflected light and detecting whether there is the table 230 above the photo sensor 284. As shown in FIG. 34, the photo sensor 284 may be located on the opposite side of the image projector 270 with respect to the center of the second gantry 150, in which case whether or not a part of the table 230 where the image projector 270 is located is outside the bore, may be detected.

The weight sensors 282-1, 282-2 may include a first weight sensor 282-1 and a second weight sensor 282-2, the first weight sensor 282-1 arranged on the same side where the image projector 270 is located with respect to the center of the table 230, and the second weight sensor 282-2 arranged on the opposite side. That is, the first weight sensor 282-1 may measure a weight on the side where the image projector 270 is located, and the second weight sensor 282-2 may measure a weight on the opposite side.

The screen controller 222 may detect a location to which the table 230 has been moved, from an intensity of the reflected light received by the photo sensor 284, and compare the measurements of the first and second weight sensors 282-1 and 282-2 to detect a position of the subject 50, such as a location where the subject 50 is lying or a location of the head of the subject 50.

The screen controller 222 may control the screen unit 210 and the image projector 270 to provide an image for the subject based on the position of the subject detected based on the detection results of the photo sensor 284 and weight sensors 282-1, 282-2.

As shown in (a) of FIG. 34, the photo sensor 284 may receive reflected light with a relatively weak intensity, i.e., it may detect absence of the table 230.

The first weight sensor 282-1 may measure a relatively heavy weight as compared with the second weight sensor 282-2. The screen controller 222 may detect from the intensity of the reflected light that a part of the table 230 where the image projector 270 is located is outside the bore, and detect from the measurements of the weight sensors 282-1, 282-2 that the head of the subject 50 is located on the same side where the image projector 270 is located.

Then, the screen controller 222 may detect that the head of the subject 50 is outside the bore, and based on this, generate a control signal to pull out the push/pull screen unit 211 and a control signal for image projection of the image projector 270. The push/pull screen unit 211 may be pulled out of the gantry 150 according to the control signal of the screen controller 150, implementing the out bore display.

As shown in (b) of FIG. 34, the photo sensor 284 may receive reflected light with a relatively weak intensity, and the second weight sensor 282-2 may measure a relatively heavy weight as compared with the first weight sensor 282-1. Based on this, the screen controller 222 may detect that the head of the subject 50 is inside the bore although a part of the table 230 is outside the bore, and generate a control signal to push in the push/pull screen unit 211 and a control signal to stop projection of the image projector 270. The push/pull screen unit 211 may be pushed into the gantry 150 according to the control signal of the screen controller 150, thus not implementing the out bore display (nor the in bore display).

As shown in (c) of FIG. 34, the photo sensor 284 may receive reflected light with a relatively strong intensity, i.e., it may detect presence of the table 230.

The first weight sensor 282-1 may measure a relatively heavy weight as compared with the second weight sensor 282-2. Based on this, the screen controller 222 may detect that the table 230 and the subject 50 are all inside the bore and the head of the subject 50 is located on the same side where the image projector 270 is located. The screen controller 222 may generate a control signal to push in the push/pull screen unit 211 and a control signal for image projection of the image projector 270. According to the control signal of the screen controller 222, the push/pull screen unit 211 may be pushed in, and thus may not implement the out bore display, but may implement the in bore display.

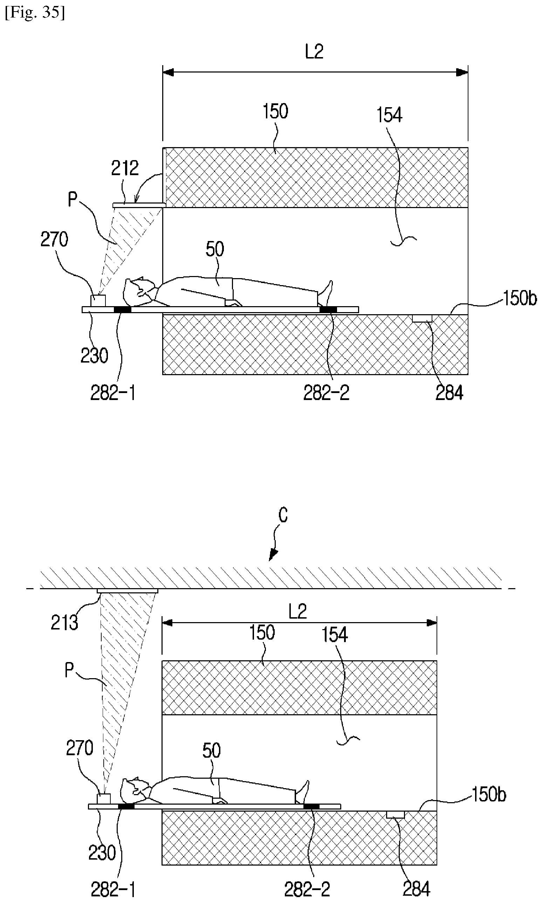

FIG. 35 shows a medical imaging apparatus including a photo sensor and weight sensors, according to an embodiment of the present disclosure.

Referring to FIG. 35, the medical imaging apparatus 1 may include a second gantry 150 with the photo sensor 284 mounted on the lower inner wall 150b, and the table 230 on which the first and second weight sensors 282-1, 282-2 are mounted. Positions or operations of the photo sensor 284 and the first and second weight sensors 282-1, 282-2 are assumed to be the same as described above in connection with FIG. 34.

Accordingly, the photo sensor 284 may receive reflected light with a relatively weak intensity, i.e., it may detect absence of the table 230. The first weight sensor 282-1 may measure a relatively heavy weight as compared with the second weight sensor 282-2. The screen controller 222 may detect from the intensity of the reflected light that a part of the table 230 where the image projector 270 is located is outside the bore, and detect from the measurements of the weight sensors 282-1, 282-2 that the head of the subject 50 is located on the same side where the image projector 270 is located. That is, the screen controller 222 may detect that the head of the subject 50 is outside the bore.

As shown in the upper diagram of FIG. 35, in the case the rotational screen unit 212 is mounted on one side of the second gantry 150, the screen controller 222 may generate a control signal to open the rotational screen unit 212 and a control signal for image projection of the image projector 270, based on the detection result. The rotational screen unit 212 may be open according to the control signal of the screen controller 150, implementing the out bore display.

As shown in the lower diagram of FIG. 35, in e case the fixed screen unit 212 is arranged outside and above the second gantry 150, the screen controller 222 may control the image projector 270 to project a content image based on the detection result. Accordingly, the fixed screen unit 213 may display the projected image, thus implementing the out bore display.

In the above description, it was assumed that the detector 280 is implemented with a table location sensor or a subject location sensor. However, the detector 280 may be implemented with an eyeball tracking sensor. The screen controller 222 may detect a location of a gaze of the subject based on the eyeball tracking sensor, and control the screen unit 210 for an image to be projected to be moved (e.g., pulled out, pushed in, or rotated) based on the detected location of the gaze of the subject, or determine a location of the screen unit 210 for the image to be projected based on the position of the subject. The screen controller 222 may also control the image projector 270 based on a change in location of the screen unit 210 corresponding to the location of the gaze of the subject. For example, the screen controller 222 may control a location for an image to be projected or a focus of the projected image for clear projection based on the change in location of the screen unit 210.

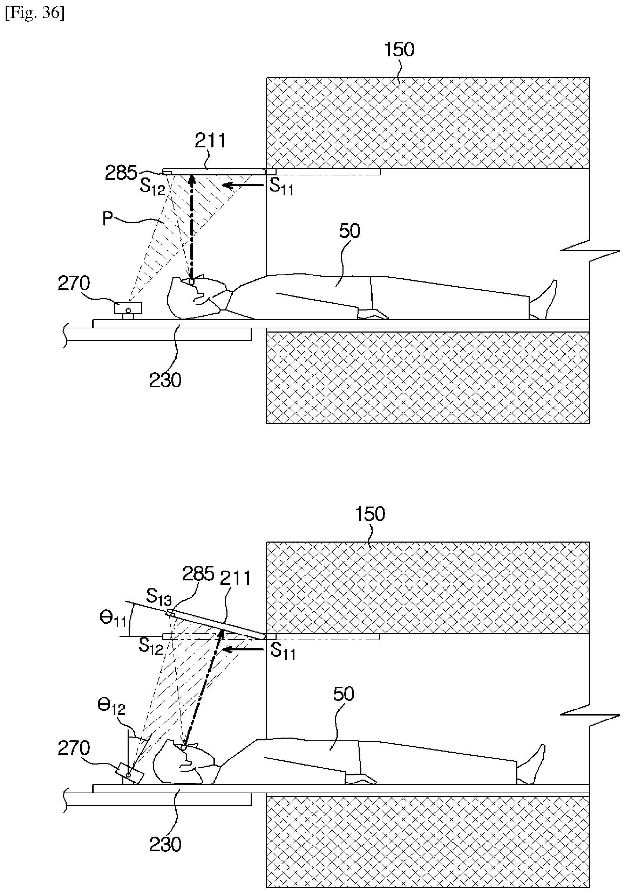

The eyeball tracking sensor (285 of FIG. 36) may be arranged on the screen unit 210 or the gantry 150, but where the eyeball tracking sensor 285 is located is not limited as long as it can detect a gaze of the subject.

Specifically, the eyeball tracking sensor 285 may be implemented with e.g., an image sensor, for capturing the face of the subject 50 and detecting locations of the eyeballs. The screen controller 222 may receive the captured image from the eyeball tracking sensor 285 and detect the gaze of the subject 50 from the locations of the eyeballs included in the captured image. The screen controller 222 may calculate a rotation angle of the screen unit 210 or image projector 270 to suit the direction of the gaze of the subject 50, and control the screen unit 210 or image projector 270 to be rotated by the calculated rotation angle.

FIG. 36 shows a medical imaging apparatus including an eyeball tracking sensor and a push/pull screen unit, according to an embodiment of the present disclosure.

The eyeball tracking sensor 285 may be mounted on the push/pull screen unit 211 for capturing the face of the subject 50, as shown in FIG. 36. The screen controller 222 may receive the captured image from the eyeball tracking sensor 285 and detect the gaze of the subject 50. The screen controller 222 may then control the push/pull screen unit 211 and the image projector 270 to project an image onto a location corresponding to the detected gaze of the subject 50.

As shown in the upper diagram of FIG. 36, if the gaze of the subject 50 is directed upward at right angles, the push/pull screen unit 211 may stay pulled out according to the control signal from the screen controller 222. That is, the push/pull screen unit 211 may be moved straight from point S11 to point S12 to receive the projected content image. As shown in the lower diagram of FIG. 36, if the gaze of the subject 50 is directed upward at an acute angle, the push/pull screen unit 211 may be pulled out and then rotated according to the control signal from the screen controller 222. Specifically, the push/pull screen unit 211 may be moved straight from point S11 to point S12, and then rotated by θ11 degrees to make a right angle with the gaze of the subject 50, i.e., rotated from the point S12 to point S13.

The screen controller 222 may control the projection focus of the image projector 270 or control the image projector 270 itself to be rotated, according to a change in position of the push/pull screen unit 211. For example, while the push/pull screen unit 211 is rotated by θ11 degrees, the image projector 270 may be rotated by θ12 degrees and project the content image.

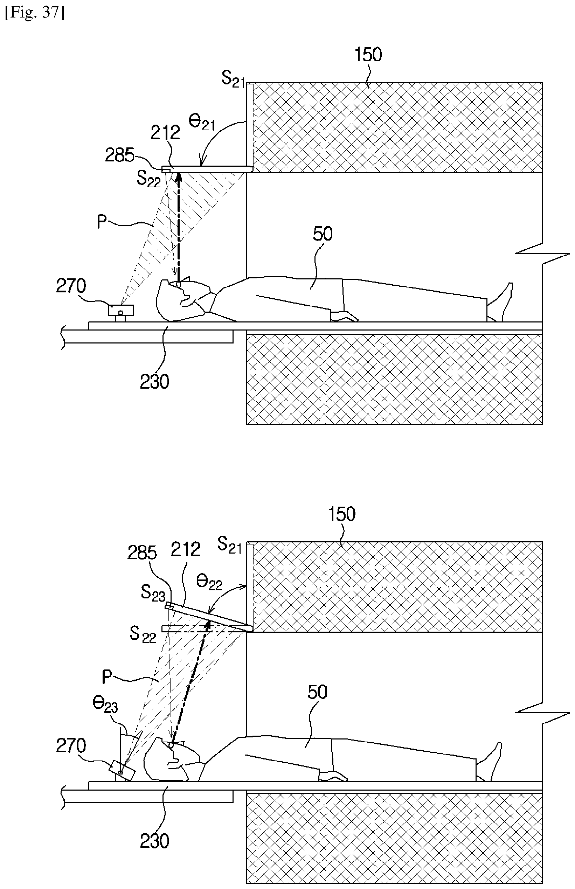

FIG. 37 shows a medical imaging apparatus including an eyeball tracking sensor and a rotational screen unit, according to an embodiment of the present disclosure.

Referring to FIG. 37, the eyeball tracking sensor 285 may be mounted on the rotational screen unit 212 for capturing the face of the subject 50. The screen controller 222 may detect the gaze of the subject 50 based on a captured image received from the eyeball tracking sensor 285, and control the rotation angle of the rotational screen unit 212 to face the gaze of the subject 50.

As shown in the upper diagram of FIG. 37, if the gaze of the subject 50 is directed upward at right angles, the rotational screen unit 212 may be rotated by θ21 degrees according to the control signal from the screen controller 222 for receiving the projected content image. As shown in the lower diagram of FIG. 37, if the gaze of the subject 50 is directed upward at an acute angle, the rotational screen unit 212 may stay at θ22 degrees according to the control signal from the screen controller 222, the θ22 being less than θ21.

The screen controller 222 may control the projection focus of the image projector 270 or control the image projector 270 itself to be rotated, according to a change in position of the rotational screen unit 212.

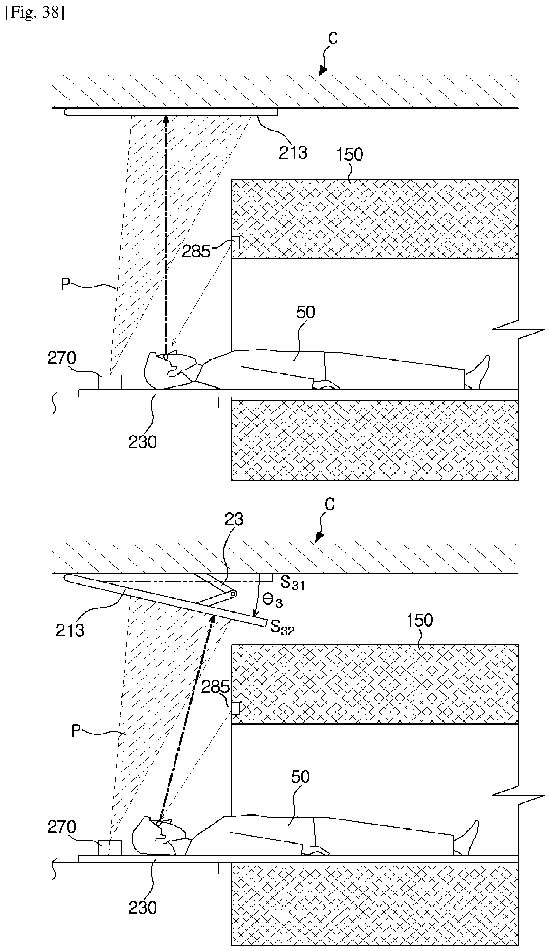

FIG. 38 shows a medical imaging apparatus including an eyeball tracking sensor and a fixed screen unit, according to an embodiment of the present disclosure.

Referring to FIG. 38, the eyeball tracking sensor 285 may be mounted on the gantry 150 for capturing the face of the subject 50, and the screen controller 22 may detect the gaze of the subject 50 based on the captured image received from the eyeball tracking sensor 285.

The fixed screen unit 213 may be rotated by means of e.g., a folding type arm 23, according to a control signal from the screen controller 222. One end of the folding type arm 23 is mounted on the ceiling C of the examination room, and the other end is fastened to the fixed screen unit 213. The fixed screen unit 213 may be rotated from the ceiling C with the arm 23 being folded or unfolded. The image projector 270 may be controlled to adjust the projection focus or to be rotated itself, to correspond to the rotation of the fixed screen unit 212.

As shown in the upper diagram of FIG. 38, if the gaze of the subject 50 is directed upward at right angles, the fixed screen unit 213 may stay fixed to the ceiling C of the examination room. As shown in the lower diagram of FIG. 38, if the gaze of the subject 50 is directed upward at an acute angle, the fixed screen unit 213 may be rotated by θ3 degrees to face the gaze of the subject 50, i.e., it may be rotated from point S12 to point S13.

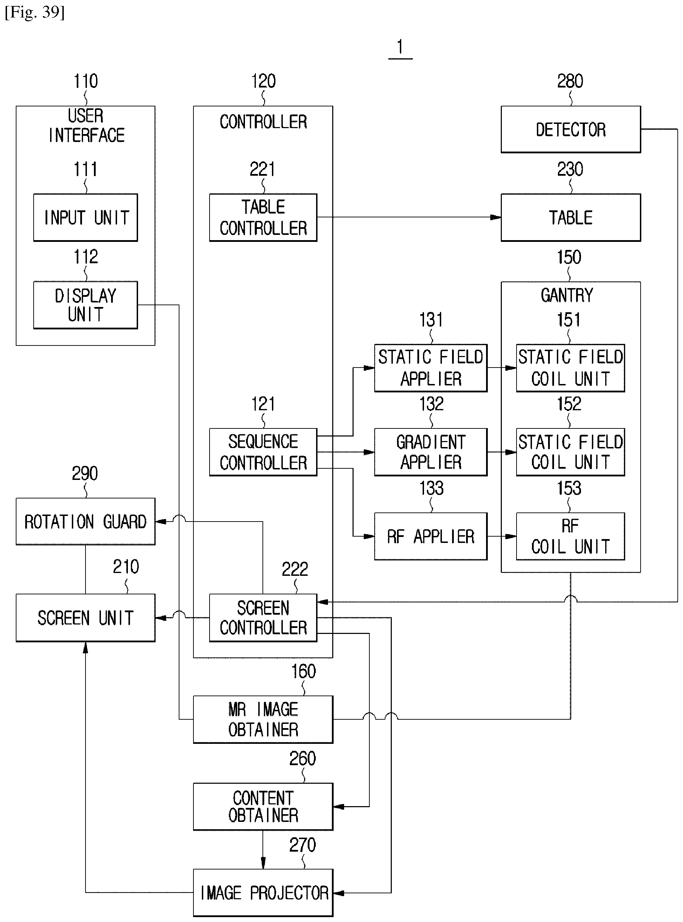

While it has been described that the screen unit 210 is rotated upward or downward to face the gaze of the subject 50 in the embodiments of FIGS. 36 to 38, the screen unit 210 may also be rotated to the left or right about the longitudinal axis of the gantry 150, which will now be described in detail in connection with FIGS. 39 to 44.

FIG. 39 is a block diagram of a medical imaging apparatus, according to another embodiment of the present disclosure.

Referring to FIG. 39, the medical imaging apparatus 1 may include the user interface 110 for providing a user interface, the table 230 for carrying a subject, the gantry 150 for producing a magnetic field and receiving a magnetic resonance signal produced from the subject, the MR image obtainer 160 for performing various processes on the magnetic resonance signal to obtain a magnetic resonance image, the content obtainer 260 for obtaining various content to be provided for the subject, the image projector 270 for projecting images contained in the content, the screen unit 210 to display the projected image, a detector 280 for detecting the position of the table or the position of the subject, a rotation guard 290 for rotating the screen unit 210, and the controller 120 for controlling overall operation of the medical imaging apparatus 1. The same components of the medical imaging apparatus 1 as described above in connection with FIG. 1 or FIG. 24 will be omitted herein.

The rotation guard 290 may be coupled with the screen unit 210 and be rotationally mounted on the front face of the gantry 150. The front face refers to a face through which the table is moved in and out. The rotation guard 290 may include e.g., a motor that enables the rotation guard 290 to be rotated about the longitudinal axis of the gantry 150 to the left or right. As the rotation guard 290 is rotated, the screen unit 290 may also be rotated.

The detector 280 may include the eyeball tracking sensor 285 implemented with e.g., an image sensor. The eyeball tracking sensor 285 may be arranged on the screen unit 210 or on the rotation guard 290, but where the eyeball tracking sensor 285 is located is not limited as long as it can detect a gaze of the subject.

The controller 120 may include the table controller 221 for controlling the movement of the table 230, the sequence controller 121 for planning a pulse sequence or controlling operation of the gantry 150 according to the pulse sequence, the content obtainer 160 for obtaining and providing content for the subject, and the screen controller 222 for controlling the image projector 270 and the screen unit 210. The screen controller 222 may receive a sensed value from the detector 280, and based on the sensed value, control the screen unit 210 and rotation of the rotation guard 290.

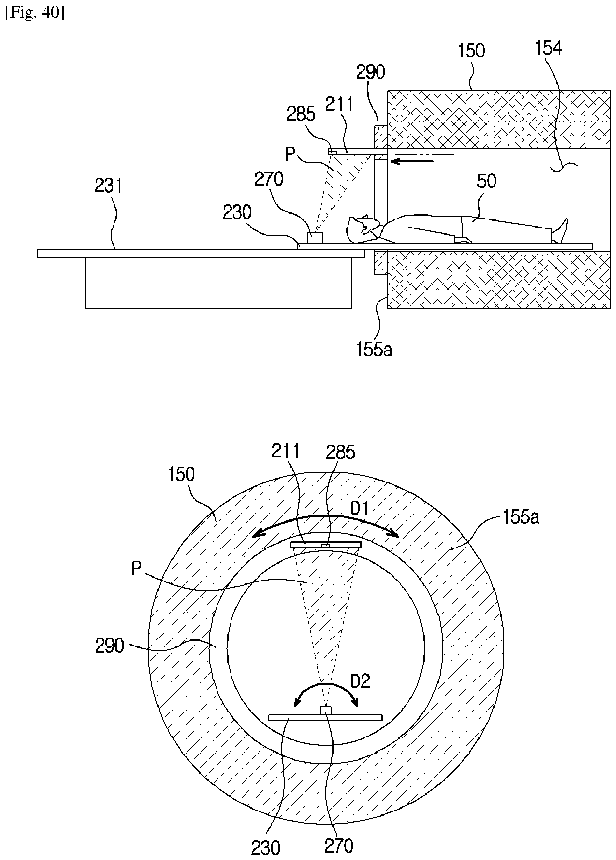

FIGS. 40 to 42 show a medical imaging apparatus including a push/pull screen unit and a rotation guard.

Referring to FIG. 40, the rotation guard 290 may be mounted on the front face 155a of the gantry 150, and have a cylindrical form to surround the bore 154. The gantry 150 may be the first gantry with the length of L1, or the second gantry with the length of L2. The rotation guard 290 may be mounted to be able to rotate to the left or right about the center in the longitudinal direction of the gantry 150, i.e., about the longitudinal axis of the gantry 150, and the rotation direction of the rotation guard 290 may be represented as direction D1.

The push/pull screen unit 211 may be pushed in or pulled out of the gantry 150 and may be formed such that it may be coupled with the rotation guard 290. For example, the push/pull screen unit 211 may be coupled with the rotation guard 290 when pulled out, and decoupled from the rotation guard 290 when pushed in. The image projector 270 may be controlled to adjust a projection focus to correspond to the rotation direction D1, and to be rotated to the left or right about the center of the table 230. The rotation direction of the image projector 270 is represented as direction D2.

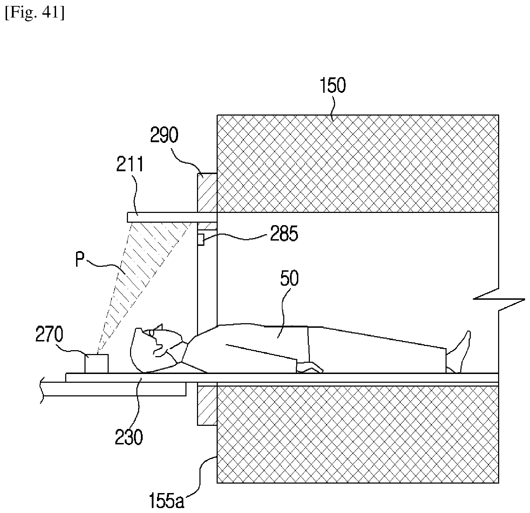

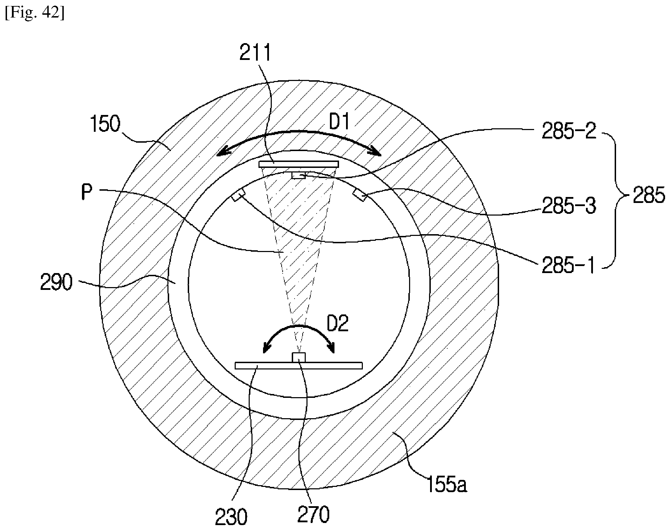

The eyeball tracking sensor 285 may be mounted on the push/pull screen unit 211 for detecting the face and gaze of the subject 50, as shown in FIG. 36. The eyeball tracking sensor 285 may also be mounted on the rotation guard 290, as shown in FIG. 41 or FIG. 42. In the case the eyeball tracking sensor 285 is mounted on the rotation guard 290, to prevent the push/pull screen unit 211 that is pulled out from interfering with scanning of the subject 50, the location of the eyeball tracking sensor 285 may be taken into account. Furthermore, the eyeball tracking sensor 285 may be implemented in plural form. For example, as shown in FIG. 42, there may be three eyeball tracking sensors 285-1, 285-2, 285-3 mounted on the rotation guard 290, centered around the push/pull screen unit 211 at intervals.

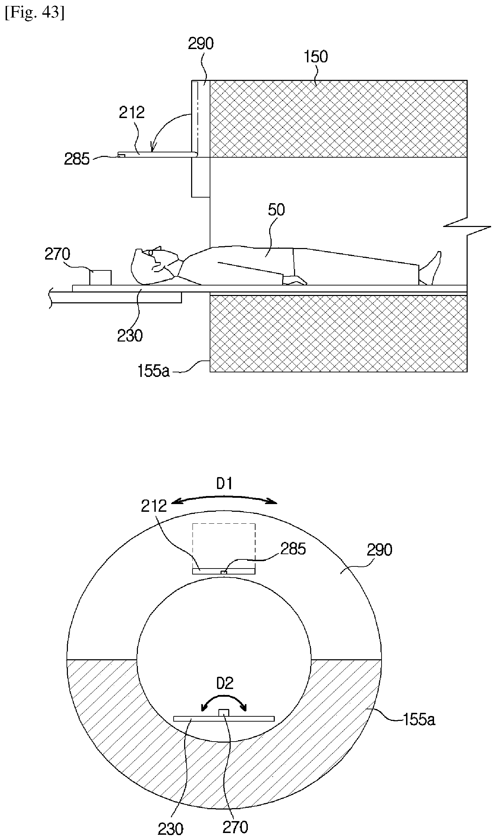

FIG. 43 shows a medical imaging apparatus including a rotational screen unit and a rotation guard, according to an embodiment of the present disclosure.

Referring to FIG. 43, the rotation guard 290 may be mounted on the front face 155a of the gantry 150 while integrated with the rotational screen unit 212 into a single unit. Specifically, the rotational screen unit 212 may be combined with the rotation guard 290 on one end, and arranged such that the other end may be rotated upward or downward about the one end as the rotation axis. The rotational screen unit 212 may remain closed by being in contact with the rotation guard 290 on one face (i.e., the reverse face to a face onto which the content image is projected), and then be changed into the open state.

While the rotation guard 290 is formed to cover a part of the front face 155a in the embodiment of FIG. 43, it may be formed to cover the entire front face 155a or may have a shape different from what is shown in FIG. 43, in other embodiments. The rotation guard 290 may be formed to be able to rotate in the direction D1, and as the rotation guard 290 is rotated, the rotational screen unit 212 combined with the rotating guard 290 may be rotated as well. The image projector 270 may be controlled to adjust the projection focus or to be rotated in the direction D2 to correspond to the rotation of the rotation guard 290 in the direction D1.

While the eyeball tracking sensor 285 may be mounted on the rotational screen unit 212, where the eyeball tracking sensor 285 is mounted is not limited as long as the eyeball tracking sensor 285 may detect the gaze of the subject.

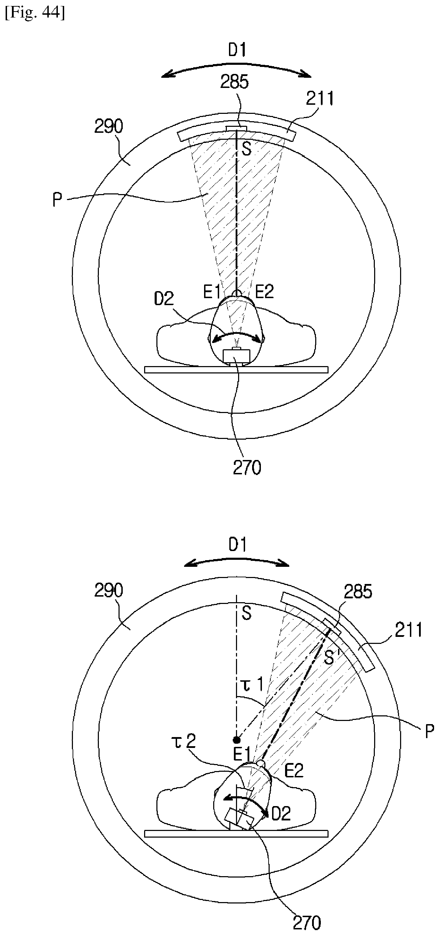

FIG. 44 shows diagrams for explaining rotation of a rotation guard and screen unit. In FIG. 44, the screen unit 210 is assumed to be implemented as the push/pull screen unit 211.

The eyeball tracking sensor 285 may be mounted on the push/pull screen unit 211 for capturing the face of the subject 50. The screen controller 222 may detect eyeball locations E1, E2 of the subject based on the captured image received from the eyeball tracking sensor 285, and detect a direction in which the face of the subject 50 is directed and a gaze direction. The screen controller 222 may also calculate a rotation angle of the rotation guard 290 or image projector 270 to correspond to the detected direction, and control the rotation guard 290 or image projector 270 to be rotated by the calculated rotation angle.

As shown in the upper diagram of FIG. 44, if the gaze S of the subject 50 is directed upward at right angles, the rotation guard 290 may remain not rotated. Accordingly, the push/pull screen unit 211 and the image projector 270 may project a content image and display the content image by reflecting off them, respectively, while remaining not rotated. On the other hand, as shown in the lower diagram of FIG. 44, if the gaze S' is directed upward at an acute angle, the rotation guard 290 may be rotated in the direction D1, and the push/pull screen unit 211 may be rotated by $\tau1$ degrees to follow the gaze direction of the subject 50. The image projector 270 may be rotated by $\tau2$ degrees to correspond to the rotation of the push/pull screen unit 211, and project a content image (P) while staying rotated.

In accordance with embodiments of the medical imaging apparatus, various contents may be provided for a person subject to scanning to relieve his/her boredom or inconvenience during the scanning process.

Several embodiments have thus been described with respect to a medical imaging apparatus, but it will be understood that various modifications can be made without departing the scope of the present disclosure. Thus, it will be apparent to those ordinary skilled in the art that the disclosure is not limited to the embodiments described, but can encompass not only the appended claims but the equivalents.

## Claims

1. A medical imaging apparatus comprising:

   a table (230), on which a subject lies and that is configured to carry the subject;
   a gantry (150), that forms an internal space and is configured to scan the subject carried into the internal space;

a screen unit, mounted on the gantry or outside the gantry; and

an image projector (270), that is configured to project an image onto the screen unit,

wherein the screen unit is configured to reflect and display the projected image,

**CHARACTERISED IN THAT**

the screen unit comprises a push/pull screen unit (211) mounted on the gantry and configured to to be pushed into the gantry or pulled out of the gantry.

2. The medical imaging apparatus of claim 1, wherein the image projector (270) is mounted on the table or a supporter for supporting the table.

3. The medical imaging apparatus of claim 1, wherein the screen unit is further mounted rotatably and/or fixedly.

4. The medical imaging apparatus of claim 1, wherein the screen unit includes the push/pull screen unit (211), a second screen unit that is mounted outside the gantry (150) and is configured to be rotated upward or downward, and a third screen unit that is arranged on the ceiling of an examination room where the gantry is located,

wherein the push/pull screen unit (211) is configured to slide into or out of the gantry, and

wherein the second screen unit is combined with the gantry on one end, and has the other end rotatable upward or downward about the one end as a rotation axis.

5. The medical imaging apparatus of claim 4, wherein the push/pull screen unit (211) is configured to be pushed into the gantry (150) if the head of the subject is located inside the gantry while scanning, wherein the push/pull screen unit is configured to be pulled out of the gantry if the head of the subject is located outside the gantry while scanning,

wherein the second screen unit is configured to be rotated at 0 degree if the head of the subject is located inside the gantry while scanning, and

wherein the second screen unit is configured to be rotated by a predetermined rotation angle if the head of the subject is located outside the gantry while scanning.

6. The medical imaging apparatus of claim 1, wherein the image projector (270) is configured to project the image if the head of the subject is located outside the gantry (150) while scanning.

7. The medical imaging apparatus of claim 4, further comprising: a first sensor that is mounted on at least one of the gantry (150) and the table (230) and is configured to detect a location of the head of the subject.

8. The medical imaging apparatus of claim7, wherein the push/pull screen unit is configured to be pulled out or pushed in, based on the detected location of the head, and

wherein the second screen unit is configured to be rotated based on the detected location of the head.

9. The medical imaging apparatus of claim 7, wherein the image projector (270) is configured to project the image based on the detected location of the head.

10. The medical imaging apparatus of claim 4, further comprising: a second sensor that is configured to detect a gaze of the subject,

wherein the second sensor is mounted on at least one of the screen unit and the gantry, and wherein the second screen unit is configured to be rotated based on the detected gaze.

11. The medical imaging apparatus of claim 9, further comprising: a rotation guard combined with the push/pull screen unit (211) or the second screen unit and rotationally mounted on the gantry (150), wherein the rotation guard is configured to be rotated to the left or right about the longitudinal axis of the gantry based on the detected gaze, and

wherein the second sensor is mounted on the rotation guard.

12. The medical imaging apparatus of claim 1, wherein the image projector (270) is configured to adjust a projection focus to face the screen unit, and wherein the image projector is configured to be rotated to face the screen unit.

13. The medical imaging apparatus of claim 1, further comprising: a detector that is configured to detect a position of the subject, and

a controller that is configured to control the screen unit and the image projector based on the position of the subject,

wherein the controller is configured to control an image projection location or image projection focus of the image projector for the image to be projected based on the position of the subject.

14. The medical imaging apparatus of claim 13, wherein the screen unit is configured to be moved in at least one way of being pushed in, pulled out, and rotated, and

wherein the controller is configured to control movement of the screen unit based on the position of the subject.

15. The medical imaging apparatus of claim 13,

wherein the screen unit includes a plurality of screens onto which an image is to be projected, and wherein the controller is configured to determine one of the plurality of screens for the image to be projected, based on the position of the subject.

**Patentansprüche**

1. Medizinische Bildgebungsvorrichtung, die umfasst:

   einen Tisch (230), auf dem ein Subjekt liegt und der zum Tragen des Subjektes konfiguriert ist; ein Gestell (150), das einen Innenraum bildet und dazu konfiguriert ist, das in den Innenraum transportierte Subjekt abzutasten; eine Bildschirmeinheit, die an dem Gestell oder außerhalb des Gestells montiert ist; und einen Bildprojektor (270), der dazu konfiguriert ist, ein Bild auf die Bildschirmeinheit zu projizieren, wobei die Bildschirmeinheit dazu konfiguriert ist, das projizierte Bild zu reflektieren und anzuzeigen,
   **DADURCH GEKENNZEICHNET, DASS**
   die Bildschirmeinheit eine zieh- bzw. drückbare Bildschirmeinheit (211) umfasst, die an dem Gestell montiert ist und dazu konfiguriert ist, in das Gestell gedrückt bzw. aus dem Gestell herausgezogen zu werden.

2. Medizinische Bildgebungsvorrichtung nach Anspruch 1, wobei der Bildprojektor (270) an dem Tisch oder an einer Trägereinrichtung zum Tragen des Tisches montiert ist.

3. Medizinische Bildgebungsvorrichtung nach Anspruch 1, wobei die Bildschirmeinheit weiterhin schwenkbar und/oder feststehend montiert ist.

4. Medizinische Bildgebungsvorrichtung nach Anspruch 1, wobei die Bildschirmeinheit Folgendes umfasst: die zieh- bzw. drückbare Bildschirmeinheit (211), eine zweite Bildschirmeinheit, die außerhalb des Gestells (150) montiert ist und dazu konfiguriert ist, nach oben oder unten geschwenkt zu werden, sowie eine dritte Bildschirmeinheit, die an der Decke des Untersuchungsraumes, in dem das Gestell sich befindet, ausgerichtet ist, wobei die zieh- bzw. drückbare Bildschirmeinheit (211) dazu konfiguriert ist, in das Gestell hinein oder aus diesem heraus zu gleiten, und wobei die zweite Bildschirmeinheit an einem Ende mit dem Gestell verbunden ist und an dem anderen Ende um das erste Ende als Schwenkachse herum nach oben oder unten schwenkbar ist.

5. Medizinische Bildgebungsvorrichtung nach Anspruch 4, wobei die zieh- bzw. drückbare Bildschirmeinheit (211) dazu konfiguriert ist, in das Gestell (150) hineingedrückt zu werden, wenn der Kopf des Subjekts sich während des Abtastvorgangs in dem Gestell befindet, wobei die zieh- bzw. drückbare Bildschirmeinheit dazu konfiguriert ist, aus dem Gestell herausgezogen zu werden, wenn der Kopf des Subjekts sich während des Abtastvorgangs außerhalb des Gestells befindet, wobei die zweite Bildschirmeinheit dazu konfiguriert ist, auf 0 (Null) Grad geschwenkt zu werden, wenn der Kopf des Subjekts sich während des Abtastvorgangs in dem Gestell befindet, und wobei die zweite Bildschirmeinheit dazu konfiguriert ist, um einen vorbestimmten Schwenkwinkel geschwenkt zu werden, wenn der Kopf des Subjekts sich während des Abtastvorgangs außerhalb des Gestells befindet.

6. Medizinische Bildgebungsvorrichtung nach Anspruch 1, wobei der Bildprojektor (270) dazu konfiguriert ist, das Bild zu projizieren, wenn der Kopf des Subjekts sich während des Abtastvorgangs außerhalb des Gestells (150) befindet.

7. Medizinische Bildgebungsvorrichtung nach Anspruch 4, die weiterhin Folgendes umfasst: einen ersten Sensor, der an dem Gestell (150) und/oder dem Tisch (230) montiert ist und dazu konfiguriert ist, eine Position des Kopfes des Subjekts zu erfassen.

8. Medizinische Bildgebungsvorrichtung nach Anspruch 7, wobei die zieh- bzw. drückbare Bildschirmeinheit dazu konfiguriert ist, auf der Basis der erfassten Position des Kopfes in das Gestell hineingedrückt bzw. aus diesem herausgezogen zu werden, und wobei die zweite Bildschirmeinheit dazu konfiguriert ist, auf der Basis der erfassten Position des Kopfes gedreht zu werden.

9. Medizinische Bildgebungsvorrichtung nach Anspruch 7, wobei der Bildprojektor (270) dazu konfiguriert ist, auf der Basis der erfassten Position des Kopfes das Bild zu projizieren.

10. Medizinische Bildgebungsvorrichtung nach Anspruch 4, die weiterhin Folgendes umfasst:

    einen zweiten Sensor, der dazu konfiguriert ist, die Blickrichtung des Subjekts zu erfassen, wobei der zweite Sensor an der Bildschirmeinheit und/oder dem Gestell montiert ist, und wobei die zweite Bildschirmeinheit dazu konfiguriert ist, auf der Basis der erfassten Blickrichtung geschwenkt zu werden.

11. Medizinische Bildgebungsvorrichtung nach Anspruch 9, die weiterhin Folgendes umfasst:

> eine mit der zieh- bzw. drückbaren Bildschirmeinheit (211) oder der zweiten Bildschirmeinheit verbundene und drehbar an dem Gestell (150) montierte drehende Frontseite,
> wobei die drehende Frontseite dazu konfiguriert ist, auf der Basis der erfassten Blickrichtung um die Längsachse des Gestells nach links oder rechts gedreht zu werden, und
> wobei der zweite Sensor an der drehenden Frontseite montiert ist.

**12.** Medizinische Bildgebungsvorrichtung nach Anspruch 1, wobei der Bildprojektor (270) dazu konfiguriert ist, einen Projektionsfokus derart einzustellen, dass er zu der Bildschirmeinheit zeigt, und wobei der Bildprojektor dazu konfiguriert ist, derart geschwenkt zu werden, dass er zu der Bildschirmeinheit zeigt.

**13.** Medizinische Bildgebungsvorrichtung nach Anspruch 1, die weiterhin Folgendes umfasst:

> eine Erfassungseinrichtung, die dazu konfiguriert ist, eine Position des Subjekts zu erfassen, und
> eine Steuerung, die dazu konfiguriert ist, die Bildschirmeinheit und den Bildprojektor auf der Basis der Position des Subjekts zu steuern,
> wobei die Steuerung dazu konfiguriert ist, eine Bildprojektionsposition oder einen Bildprojektionsfokus des Bildprojektors derart zu steuern, dass das Bild auf der Basis der Position des Subjekts projiziert wird.

**14.** Medizinische Bildgebungsvorrichtung nach Anspruch 13, wobei die Bildschirmeinheit dazu konfiguriert ist, bewegt zu werden, indem sie hineingedrückt, herausgezogen und/oder geschwenkt wird, und wobei die Steuerung dazu konfiguriert ist, die Bewegung der Bildschirmeinheit auf der Basis der Position des Kopfes zu steuern.

**15.** Medizinische Bildgebungsvorrichtung nach Anspruch 13, wobei die Bildschirmeinheit eine Mehrzahl von Bildschirmen, auf die ein Bild projiziert werden soll, umfasst, und wobei die Steuerung dazu konfiguriert ist, auf der Basis der Position des Subjekts einen aus der Mehrzahl von Bildschirmen für das zu projizierende Bild zu bestimmen.

**Revendications**

**1.** Dispositif d'imagerie médicale comprenant :

> une table (230) sur laquelle est allongé un sujet qu'elle est conçue pour porter,
> un portique (150) qui délimite un espace interne et est conçu pour effectuer un examen par imagerie du sujet transporté dans l'espace interne,
> une unité d'écran montée sur le portique ou en dehors du portique, et
> un projecteur d'image (270) qui est conçu pour projeter une image sur l'unité d'écran ;
> ladite unité d'écran étant conçue pour réfléchir et présenter l'image projetée,
> **CARACTÉRISÉ EN CE QUE**
> l'unité d'écran comprend une unité d'écran escamotable (211) montée sur le portique et conçue pour être repoussée dans le portique ou tirée hors du portique.

**2.** Dispositif d'imagerie médicale selon la revendication 1, dans lequel le projecteur d'image (270) est monté sur la table ou sur un support visant à supporter la table.

**3.** Dispositif d'imagerie médicale selon la revendication 1, dans lequel l'unité d'écran est en outre montée rotative et/ou fixe.

**4.** Dispositif d'imagerie médicale selon la revendication 1, dans lequel l'unité d'écran comporte l'unité d'écran escamotable (211), une deuxième unité d'écran montée en dehors du portique (150) et conçue pour pivoter vers le haut ou vers le bas, et une troisième unité d'écran agencée au plafond de la salle d'examen dans laquelle est situé le portique,
dans lequel l'unité d'écran escamotable (211) est conçue pour entrer et sortir du portique par coulissement, et
dans lequel la deuxième unité d'écran est associée au portique à une extrémité et peut pivoter, à l'autre extrémité, vers le haut ou vers le bas par rapport à ladite une extrémité qui sert d'axe de pivotement.

**5.** Dispositif d'imagerie médicale selon la revendication 4, dans lequel l'unité d'écran escamotable (211) est conçue pour être repoussée dans le portique (150) si la tête du sujet est située à l'intérieur du portique pendant l'examen par imagerie,
dans lequel l'unité d'écran escamotable est conçue pour être tirée hors du portique si la tête du sujet est située à l'extérieur du portique pendant l'examen par imagerie,
dans lequel la deuxième unité d'écran est conçue pour pivoter à 0 degré si la tête du sujet est située à l'intérieur du portique pendant l'examen par imagerie, et
dans lequel la deuxième unité d'écran est conçue pour pivoter selon un angle de pivotement prédéterminé si la tête du sujet est située à l'extérieur du portique pendant l'examen par imagerie.

**6.** Dispositif d'imagerie médicale selon la revendication 1, dans lequel le projecteur d'image (270) est conçu pour projeter l'image si la tête du sujet est située à l'extérieur du portique (150) pendant l'examen par imagerie.

**7.** Dispositif d'imagerie médicale selon la revendication 4, comprenant en outre :
un premier capteur monté sur le portique (150) et/ou la table (230) et conçu pour détecter l'emplacement de la tête du sujet.

**8.** Dispositif d'imagerie médicale selon la revendication 7, dans lequel l'unité d'écran escamotable est conçue pour être tirée ou repoussée en fonction de l'emplacement détecté de la tête, et
dans lequel la deuxième unité d'écran est conçue pour pivoter en fonction de l'emplacement détecté de la tête.

**9.** Dispositif d'imagerie médicale selon la revendication 7, dans lequel le projecteur d'image (270) est conçu pour projeter l'image en fonction de l'emplacement détecté de la tête.

**10.** Dispositif d'imagerie médicale selon la revendication 4, comprenant en outre :

un deuxième capteur conçu pour détecter le regard du sujet,
dans lequel le deuxième capteur est monté sur l'unité d'écran et/ou le portique, et
dans lequel la deuxième unité d'écran est conçue pour pivoter en fonction du regard détecté.

**11.** Dispositif d'imagerie médicale selon la revendication 9, comprenant en outre :

un fronton rotatif associé à l'unité d'écran escamotable (211) ou à la deuxième unité d'écran et monté rotatif sur le portique (150),
dans lequel le fronton rotatif est conçu pour subir une rotation à gauche ou à droite sur l'axe longitudinal du portique en fonction du regard détecté, et
dans lequel le deuxième capteur est monté sur le fronton rotatif.

**12.** Dispositif d'imagerie médicale selon la revendication 1, dans lequel le projecteur d'image (270) est conçu pour régler le foyer de projection afin qu'il soit dirigé vers l'unité d'écran, et dans lequel le projecteur d'image est conçu pour pivoter de façon à être dirigé vers l'unité d'écran.

**13.** Dispositif d'imagerie médicale selon la revendication 1, comprenant en outre : un détecteur conçu pour détecter la position du sujet, et

un organe de commande conçu pour commander l'unité d'écran et le projecteur d'image en fonction de la position du sujet,
dans lequel l'organe de commande est conçu pour commander l'emplacement de la projection d'image ou le foyer de projection d'image du projecteur d'image afin que l'image soit projetée en fonction de la position du sujet.

**14.** Dispositif d'imagerie médicale selon la revendication 13, dans lequel l'unité d'écran est conçue pour subir un mouvement en étant repoussée, tirée et/ou pivotée, et
dans lequel l'organe de commande est conçu pour contrôler le mouvement de l'unité d'écran en fonction de la position du sujet.

**15.** Dispositif d'imagerie médicale selon la revendication 13, dans lequel l'unité d'écran comporte une pluralité d'écrans sur lesquels une image est destinée à être projetée, et
dans lequel l'organe de commande est conçu pour déterminer un écran parmi la pluralité d'écrans sur lequel l'image doit être projetée en fonction de la position du sujet.

[Fig. 1]

1

[Fig. 2a]

[Fig. 2b]

[Fig. 3a]

[Fig. 3b]

[Fig. 4]

[Fig. 5]

[Fig. 6]

[Fig. 7]

379

271

230

50

231

(a)

50

230

271

231

(b)

271

230

71a

(c)

[Fig. 8]

[Fig. 9]

[Fig. 10]

EP 3 212 079 B1

37

[Fig. 11]

[Fig. 12]

(a)

(b)

(c)

[Fig. 13]

[Fig. 14]

[Fig. 15]

[Fig. 16]

[Fig. 17]

[Fig. 18]

L1

150   154

212

P

231

270-1

230

50

270-2

L1

154   150

212

270-1

150a

230

50

P

231

270-2

[Fig. 19]

[Fig. 20]

[Fig. 21]

[Fig. 22]

[Fig. 23]

[Fig. 24]

[Fig. 25]

[Fig. 26]

[Fig. 27]

[Fig. 28]

[Fig. 29]

[Fig. 30]

(a)

(b)

(c)

[Fig. 31]

[Fig. 32]

(a)

(b)

(c)

[Fig. 33]

[Fig. 34]

(a)

(b)

(c)

[Fig. 35]

[Fig. 36]

[Fig. 37]

[Fig. 38]

[Fig. 39]

[Fig. 40]

[Fig. 41]

[Fig. 42]

[Fig. 43]

[Fig. 44]

**EP 3 212 079 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013208249 A
- US 2012143040 A
- US 2014125337 A
- US 5143373 A